# EUROPÄISCHE PATENTANMELDUNG

(11) **EP 4 552 547 A1**
(43) Veröffentlichungstag der Anmeldung: **14.05.2025**
(21) Anmeldenummer: 24211183.9
(22) Anmeldetag: 06.11.2024
(51) Int. Cl.: A61B 1/00, A61B 1/04, G02B 7/00, G02B 23/24

(54) **FILTERWECHSELVORRICHTUNG FÜR EINE ENDOSKOPISCHE KAMERA, KAMERAKOPF FÜR EIN ENDOSKOP UND NACHRÜSTSATZ ZUM NACHRÜSTEN EINES KAMERAKOPFES UND/ODER EINES ENDOSKOPS**

(30) Priorität: 07.11.2023 DE 102023130857
(71) Anmelder: Karl Storz SE & Co. KG, 78532 Tuttlingen (DE)
(72) Erfinder: Huber, Florian, 78532 Tuttlingen (DE); Kroll, Kai, 78532 Tuttlingen (DE)

(57) **Zusammenfassung**

Die Erfindung betrifft eine Filterwechselvorrichtung für eine endoskopische Kamera, einen Kamerakopf und einen Nachrüstsatz für einen Kamerakopf und/oder Endoskop. Die Filterwechselvorrichtung weist eine Grundplatte, eine relativ zur Grundplatte drehbare Filterplatte, einen optischen Durchgang mit einer optischen Achse und mindestens zwei schwenkbare Filterhalter mit jeweils mindestens einem optischen Filter auf. Die Filterplatte weist eine Führungsnut mit einer umlaufenden Führungsbahn auf. Die Filterhalter sind jeweils drehbar und weisen ein zumindest teilweise in der Führungsnut angeordnetes Führungselement auf. Die Führungsnut weist einen konzentrisch zur optischen Achse ausgebildeten Abschnitt und einen nichtkonzentrisch zur optischen Achse ausgebildeten Abschnitt auf. Bei Anordnung mindestens eines Führungselements in dem konzentrischen Abschnitt ist der jeweilige Filterhalter frei von dem optischen Durchgang angeordnet. Der nicht-konzentrische Abschnitt weist einen ersten Teilabschnitt mit einer maximal beabstandeten Führungsnutposition auf, welcher einen größeren Abstand zur optischen Achse als der konzentrische Abschnitt aufweist. Bei Anordnung des jeweiligen Führungselementes in der maximal beabstandeten Führungsnutposition, ist der jeweilige Filterhalter in den optischen Durchgang eingeschwenkt.

## Beschreibung

Die Erfindung betrifft eine Filterwechselvorrichtung für eine endoskopische Kamera, wobei die Filterwechselvorrichtung eine Grundplatte, eine drehbare Filterplatte, einen optischen Durchgang mit einer optischen Achse und mindestens zwei schwenkbare Filterhalter mit jeweils mindestens einer Aufnahme für ein optisches Filter aufweist, wobei die drehbare Filterplatte eine Führungsnut mit einer umlaufenden Führungsbahn aufweist und relativ zur Grundplatte drehbar ist, die Filterhalter jeweils mittels einer Rotationsachse drehbar auf der Grundplatte angeordnet sind und jeweils ein Führungselement aufweisen, wobei das jeweilige Führungselement zumindest teilweise in der Führungsnut angeordnet ist. Des Weiteren betrifft die Erfindung einen Kamerakopf für ein Endoskop und einen Nachrüstsatz zum Nachrüsten eines Kamerakopfes und/oder eines Endoskopes.

In medizinischen und nicht-medizinischen Anwendungen werden Beobachtungsinstrumente wie Endoskope eingesetzt, um innere Hohlräume eines menschlichen oder tierischen Körpers oder eines industriellen, technischen Objektes, wie eine Rohrleitung, zu untersuchen. Für die Bildgebung kann ein Kamerakopf aufweisend einen Bildsensor zusammen mit dem Endoskop eingesetzt werden. Um die Bildqualität zu verbessern und/oder verschiedene Beobachtungsmodi zu ermöglichen, ist es bekannt, verschiedene Filter in den Strahlengang des Beobachtungsinstrumentes einzubringen.

Zum Beispiel wird in der Fluoreszenz-Bildgebung das zu untersuchende Objekt einem Licht mit einer Anregungsstrahlung ausgesetzt, welche ein vorher auf das Objekt aufgebrachtes oder bereits vorhandenes Fluorophor anregt, Licht einer bestimmten Emissionswellenlänge auszusenden, wobei die Anregungswellenlänge und die Emissionswellenlänge sich üblicherweise unterscheiden. Normalerweise ist die Emissionswellenlänge länger als die Anregungswellenlänge. Das abgegebene Emissionslicht ist üblicherweise deutlich schwächer als andere Lichtquellen, wie das Anregungsfluoreszenzlicht oder das bildgebende Weißlicht. Aus diesen Gründen ist es notwendig, unerwünschte Wellenlängenbänder mittels eines Filters herauszufiltern, sodass möglichst nur das gewünschte Spektrum und/oder die Emissionswellenlänge des Fluorophors den Kamerakopf im Fluoreszenzmodus erreicht. Zum Wechsel zwischen verschiedenen Beobachtungsmodi werden üblicherweise zwei oder mehrere Filter nacheinander in den Strahlengang der Beobachtungsoptik eingebracht, wozu prinzipiell verschiedene Filterwechsler bekannt sind.

DE 101 57 075 A1 offenbart eine Vorrichtung zum Positionieren zumindest eines optischen Bauelementes innerhalb eines endoskopischen Systems mit einem Gehäuse, durch welches die optische Achse des endoskopischen Systems verläuft und in dem das zumindest eine Bauelement angeordnet ist, welches um eine im Wesentlichen parallel zu einer Längsachse des Gehäuses verlaufenden Schwenkachse in den Strahlengang einschwenkbar und aus diesem wieder ausschwenkbar ist, wobei das zumindest eine Bauelement, beispielsweise ein Filter für einen speziellen spektralen Wellenlängenbereich, an einem um die Schwenkachse schwenkbaren Träger angeordnet ist. Hierbei ist ein kleinster Abstand einer Innenwand des Gehäuses von der Schwenkachse kleiner als ein größter Abstand der Schwenkachse zu einer Außenkante des zumindest einen Bauelementes. Durch die nähere Anordnung der Schwenkachse jedes schwenkbaren Trägers zu der Innenwand des Gehäuses ist die Anzahl der Träger zum Halten möglichst unterschiedlicher Filter durch die räumlich weitreichende Schwenkbewegung innerhalb des limitierten Raumes des Gehäuses begrenzt. Zudem ist das Gehäuse fest mit einem Gehäuse des optischen Kopfes des Endoskopes verbunden. Weiterhin nachteilig ist, dass durch räumlich nähere Anordnung der Schwenkachse an der Innenwand des Gehäuses eine Vielzahl von beweglichen Einzelteilen erforderlich ist, um mehrere Filter in den Strahlengang einzubringen. Dies hat einen erhöhten Kosten- und Montageaufwand zur Folge. Zudem erhöht sich dadurch das Risiko von Verschleiß, Ungenauigkeiten in der jeweiligen Schwenkbewegung und folglich von Fehlfunktionen.

Aus dem anmeldereigenen Stand der Technik der DE 10 2022 131 502 A1 ist eine Filterwechselvorrichtung für einen endoskopischen Kamerakopf mit mindestens drei optischen Filtern bekannt, welche eine Nut mit einer nicht kreisförmigen Führungsbahn und einem Drehelement aufweist, wobei durch Drehen des Drehelementes nacheinander in der Nut angeordnete Träger für jeweils ein Filter bewegt oder eine Schwenkbewegung eines Trägerarms mittels eines partiell in der Nut angeordneten Führungselementes aufgrund einer zumindest partiellen Bewegung entlang der nicht kreisförmigen Führungsbahn in und aus dem Strahlengang erfolgt. Nachteilig hierbei ist, dass zur Anordnung von mehreren Filtern jeweils ein Trägerarm mit einem Führungselement notwendig ist, sodass es aufgrund der größeren Anzahl von Führungselementen oder direkt in der Nut angeordneten Filterträgern aufgrund der auftretenden Reibungskräfte zu einem Verschleiß und/oder störenden Einflüssen auf die Funktion der Filterwechselvorrichtung kommen kann. Zudem überlappen sich die schwenkbaren Trägerarme, sodass entlang der optischen Achse ein größerer Bauraum notwendig ist.

Die US 2 684 611 A beschreibt eine Halterung für photographische Objektive, bei welcher vier Filter endständig jeweils von einem Filterträger in Form eines Kniehebels gehalten werden, wobei ein jeweils kürzere Arm der Filterträger einen gelagerten Drehzapfen und einen Führungszapfen aufweist und die Führungszapfen in einer gebogenen Führungsnut eines Filtereinstellrings drehbar angeordnet sind. Die gebogene Führungsnut weist über den Großteil ihrer Länge die Form eines Kreises konzentrisch zur Achse der Objektivhalterung und einen kürzeren nach innen gebogenen, abgeflachten Abschnitt auf. Bei Anordnung eines Führungszapfen in der Mitte des nach innen gerichteten, abgeflachten Abschnittes wird der zugehörige Filterträger nach innen geschwenkt und sein endständiges Filter in einer Position konzentrisch zur optischen Achse des Objektives angeordnet.

Aufgabe der Erfindung ist es, den Stand der Technik zu verbessern. Insbesondere ist es eine Aufgabe der Erfindung eine Filterwechselvorrichtung für eine endoskopische Kamera, einen Kamerakopf für ein Endoskop und eine Nachrüsteinheit bereitzustellen, welche die räumliche Ausnutzung der Vorrichtung verbessern, die bauliche Konstruktion vereinfachen, einen zuverlässigen Filterwechsel ermöglichen, einen Verschleiß beim Filterwechsel vermindern und ein schnelles, präzises und effizientes Wechseln von Filtern erlaubt.

Gelöst wird die Aufgabe durch eine Filterwechselvorrichtung für eine endoskopische Kamera, wobei die Filterwechselvorrichtung eine Grundplatte, eine drehbare Filterplatte, einen optischen Durchgang mit einer optischen Achse und mindestens zwei schwenkbare Filterhalter mit jeweils mindestens einer Aufnahme für ein optisches Filter aufweist, wobei der optische Durchgang um einen Mittelpunkt eines Querschnittes der drehbaren Filterplatte angeordnet ist und die drehbare Filterplatte eine Führungsnut mit einer umlaufenden Führungsbahn aufweist und relativ zur Grundplatte drehbar ist, die Filterhalter jeweils mittels einer Rotationsachse drehbar auf der Grundplatte angeordnet sind und jeweils ein Führungselement aufweisen, wobei das jeweilige Führungselement zumindest teilweise in der Führungsnut angeordnet ist und die Führungsnut einen ersten konzentrisch zur optischen Achse ausgebildeten Abschnitt und einen zweiten nicht-konzentrisch zur optischen Achse ausgebildeten Abschnitt aufweist, sodass bei einer Anordnung mindestens eines Führungselementes in dem ersten konzentrisch ausgebildeten Abschnitt der jeweilige Filterhalter in einer Ausgangsposition frei von dem optischen Durchgang angeordnet ist. Bei einer Anordnung mindestens eines Führungselementes in dem zweiten nicht-konzentrisch ausgebildeten Abschnitt ist der zugehörige Filterhalter in den optischen Durchgang eingeschwenkt. Vorteilhaft weist der zweite nicht-konzentrisch ausgebildete Abschnitt der Führungsnut einen ersten Teilabschnitt mit einer maximal beabstandeten Führungsnutposition auf, welcher einen größeren Abstand zur optischen Achse als der erste konzentrisch ausgebildete Abschnitt aufweist, sodass bei Anordnung des jeweiligen Führungselementes an der maximal beabstandeten Führungsnutposition der jeweilige Filterhalter in den optischen Durchgang eingeschwenkt ist.

Somit ist eine Filterwechselvorrichtung bereitgestellt, mit welcher zwei oder mehrere Filter in den Strahlengang einer endoskopischen Kamera präzise und effizient ein- und ausschwenkbar sind.

Es ist besonders vorteilhaft, dass durch die Geometrie und den Verlauf der Führungsnut mit einem ersten konzentrisch ausgebildeten Abschnitt und einem zweiten nicht-konzentrisch ausgebildeten Abschnitt eine definierte Ausschwenk-, Einschwenk-, Zwischen-, Ausgangs- und/oder Ruheposition des jeweiligen Filterhalters vorgegeben ist, wodurch der jeweilige schwenkbare Filterhalter oder die schwenkbaren Filterhalter präzise und effizient nutzbar sind. Neben einer Festlegung einer definierten Ausrichtung und/oder Position des jeweiligen Filterhalters zur optischen Achse und/oder dem Durchgang aufgrund der Geometrie und dem Verlauf der Führungsnut und der Positionierung des jeweiligen Führungselementes in der Führungsnut wird das Führungselement in der Führungsnut gehalten, sodass selbst bei einem Verschleiß und somit einem Spiel der Rotationsachse des jeweiligen Filterhalters dennoch die vordefinierte Ausschwenk-, Einschwenk-, Zwischen-, Ausgangs- und/oder Ruheposition des jeweiligen Filterhalters eingehalten wird. Folglich wird über die Rotationsachse zwar eine Schwenkbewegung des jeweiligen Filterhalters initiiert, das genaue Einhalten der gewünschten Position des jeweiligen Filterhalters wird jedoch im Zusammenwirken der Führungsbahn mit dem Führungselement des Filterhalters vorgegeben. Dadurch wird das präzise Einnehmen und/oder Einhalten der gewünschten Position gewährleistet. Somit werden je nach Position des jeweiligen Führungselementes in dem ersten konzentrisch ausgebildeten Abschnitt oder dem zweiten nicht-konzentrisch ausgebildeten Abschnitt aufgrund der Ausbildung, der Form und dem Verlauf dieser beiden Abschnitte definierte, aufeinander folgende lokale Positionen (auch Führungsnutpositionen genannt) für das jeweilige Führungselement in der Führungsnut vorgegeben, an welchen der zugehörige Filterhalter eine Ausschwenk-, Einschwenk-, Zwischen-, Ausgangs- und/oder Ruheposition einhält und/oder einnimmt und sich in einer entsprechenden räumlichen Ausrichtung zum optischen Durchgang befindet. Somit wird durch den Verlauf der Führungsnut die Reihenfolge und der zeitliche Ablauf der Wechsel der Filter vorgegeben.

Dadurch ist oder wird bei Erreichen des sich in der Führungsnut bewegenden jeweiligen Führungselementes an einer vorgegebenen örtlichen Position und/oder Führungsnutposition des zweiten nicht-konzentrisch ausgebildeten Abschnittes automatisch eine Einschwenk- oder Ausschwenkbewegung des jeweiligen Filterhalters realisiert.

Dadurch, dass die Führungsnut rundumlaufend ausgebildet ist und folglich das Ende des ersten konzentrisch ausgebildeten Abschnittes in den Anfang des nicht-konzentrisch ausgebildeten Abschnittes und das Ende des nicht-konzentrisch ausgebildeten Abschnittes in den Anfang des ersten konzentrisch ausgebildeten Abschnittes übergehen, kann das jeweilige Führungselement unbegrenzt in der Führungsnut rundumlaufen. Folglich kann die drehbare Filterplatte beständig vom Anwender durchrotiert werden.

Zudem ermöglicht der Verlauf der Führungsnut mit dem ersten konzentrisch ausgebildeten Abschnitt und dem zweiten nicht-konzentrisch ausgebildeten Abschnitt aufgrund ihrer Form und der dadurch vorgegebenen definierten Positionen, an denen der zugehörige Filterhalter in der Ausgangsposition verbleibt oder eine bestimmte vorgegebene Bewegung durchführt, dass die Positionen und die Bewegungen der mindestens zwei schwenkbaren Filterhalter optimal zeitlich und räumlich aufeinander abgestimmt sind. Dadurch ist es ermöglicht, dass die mindestens zwei Filterhalter in einer Ebene und/oder nebeneinander angeordnet sind. Folglich wird durch die aufeinander abgestimmten räumlichen Anordnungen und Bewegungen der Filterhalter und durch eine kompakte Bauform der Filterhalter selbst eine sehr kleine Bauform der Filterwechselvorrichtung ermöglicht. Vor allem weist die Filterwechselvorrichtung aufgrund der Anordnung der mindestens zwei Filterhalter in einer Ebene eine geringe Abmessung entlang der optischen Achse auf.

Neben der Form und dem Verlauf der Führungsnut mit dem ersten konzentrisch ausgebildeten Abschnitt und dem zweiten nicht-konzentrisch ausgebildeten Abschnitt und somit der jeweiligen Position des jeweiligen Führungselementes innerhalb der Führungsnut, sind die mindestens zwei Filterhalter mittels ihrer jeweiligen Rotationsachse in einem vorgegebenen räumlichen Abstand und in einer räumlichen Position zueinander auf der Grundplatte angeordnet. Dadurch ist die räumliche Anordnung des ersten Führungselementes und des zweiten Führungselementes und deren Abstand entlang der Führungsbahn vorgegeben. Befindet sich beispielsweise das erste Führungselement innerhalb des ersten konzentrisch ausgebildeten Abschnittes und liegt der erste Filterhalter damit in der Ausgangsposition frei von dem Durchgang vor, so ist das zweite Führungselement innerhalb des zweiten nicht-konzentrisch ausgebildeten Abschnittes angeordnet und führt je nach der lokalen Position entlang des Verlaufs des zweiten nicht-konzentrisch ausgebildeten Abschnittes eine entsprechende Bewegung aus oder nimmt eine Zwischen- oder Ausgangsposition ein.

Ein wesentlicher Gedanke der Erfindung beruht darauf, eine Führungsnut in einer drehbaren Filterplatte mit einer umlaufenden Führungsbahn gezielt mit einem ersten konzentrisch zur optischen Achse ausgebildeten Abschnitt und einem zweiten nicht-konzentrisch zur optischen Achse ausgebildeten Abschnitt auszubilden, wobei aufgrund der jeweiligen Form und des Verlaufes der konzentrischen und nicht-konzentrischen Abschnitte bei einem Drehen der Filterplatte ein Führungselement eines jeweiligen Filterhalters in und entlang der Führungsnut bewegt wird, wodurch bei Erreichen des Führungselementes einer jeweils vorgegebenen Position und/oder Führungsnutposition in dem konzentrischen oder dem nicht-konzentrischen Abschnitt, dem zugehörigen Filterhalter eine Ausgangs-, Zwischen- und/oder Ruheposition oder eine gezielte Bewegung zum Einschwenken in oder Ausschwenken aus dem optischen Durchgang aufgeprägt wird. Durch den Verlauf des konzentrischen Abschnitts und des nicht-konzentrischen Abschnittes werden verschiedene, aufeinanderfolgende Führungsnutpositionen für die Führungselemente und dadurch bedingt eine räumliche Ausrichtung des jeweiligen Filterhalters zu dem optischen Durchgang vorgegeben. Im Zusammenwirken mit der drehbaren Anordnung der mindestens zwei Filterhalter auf der Grundplatte mittels einer jeweiligen Rotationsachse wird ein zeitlicher Ablauf und eine Reihenfolge des Wechsels der Filter von der jeweiligen Führungsnutposition des Führungselementes in der Führungsnut vorgegeben. Somit wird eine Filterwechselvorrichtung bereitgestellt, mit welcher verschiedene Filter, insbesondere Fluoreszenzfilter, in den Strahlengang einer endoskopischen Kamera präzise, schnell, effizient und mit einer langen Standzeit ein- und ausschwenkbar sind.

Folgendes Begriffliche sei erläutert:

Eine "Filterwechselvorrichtung" ist insbesondere eine Vorrichtung, mit welcher einer von zwei oder mehreren Filtern in und aus dem optischen Strahlengang bewegbar ist. Die Filterwechselvorrichtung ist insbesondere durch Drehen der drehbaren Filterplatte manuell oder automatisch zum Wechseln der Filter aktivierbar. Hierbei kann ein Wechsel zwischen zwei Filteraufnahmen und/oder Filtern eines einzigen Filterhalters oder zwischen zwei Filteraufnahmen und/oder Filtern zweier Filterhalter erfolgen. Die Filterwechselvorrichtung weist insbesondere mindestens zwei optische Filter, bevorzugt mindestens drei optische Filter, auf. Optional kann die Filterwechselvorrichtung auch eine Filteraufnahme aufweisen, welche kein optisches Filter aufweist und somit einen freien Durchlass durch den optischen Strahlengang ermöglicht. Somit kann mittels der Filterwechselvorrichtung auch eine leere Filteraufnahme in den optischen Durchgang und in den Strahlengang eingebracht werden. Ebenso kann der freie Durchgang statt Weglassen eines optischen Filters auch durch ein nicht filterndes optisches Element, wie eine Glasscheibe, ermöglicht sein. Eine Glasscheibe als Fenster kann auch eine Anti-Reflexions-Beschichtung aufweisen. Die Filterwechselvorrichtung kann insbesondere in einer Kamera integriert sein oder als separate Vorrichtung, beispielsweise als Aufschnappfilter ausgebildet und mit der Kamera und/oder einem Endoskop verbindbar sein.

Ein "optisches Filter" (vereinfacht auch als "Filter" bezeichnet) ist insbesondere ein optisches Element, welches die einfallende Strahlung und/oder einfallenden Strahlen basierend auf spezifischen Eigenschaften, wie beispielsweise einer Wellenlänge, einem Polarisationszustand, einem Einfallswinkel und/oder einer Einfallsrichtung, selektiert und somit durchlässt oder am Durchlassen hindert. Ebenso kann ein optisches Filter die Eigenschaften des durchtretenden Lichtes verändern, beispielsweise durch Umwandlung von kreisförmig polarisiertem Licht in linearpolarisiertes Licht. Insbesondere kann ein optisches Filter ein spezifisches spektrales Wellenlängenband blockieren. Ein optisches Filter kann beispielsweise ein Verlaufsfilter, ein Kantenfilter, ein Polarisationsfilter oder ein Interferenzfilter sein. Ein Interferenzfilter weist insbesondere eine Beschichtung auf, welche ein Blockieren oder Durchlassen von Licht eines bestimmten Spektralbereiches bewirkt. Das optische Filter ist insbesondere als Beobachtungsfilter und/oder Detektionsfilter, Fluoreszenzbeobachtungsfilter oder Anregungsfilter einsetzbar. Ein Fluoreszenzbeobachtungsfilter (auch als Fluoreszenzfilter bezeichnet) ist insbesondere ein optisches polychroitisches Interferenzfilter zum Trennen des emittierten Fluoreszenzlichtes von dem eingesetzten Anregungslicht. Somit blockiert das Fluoreszenzfilter die spezifische Fluoreszenzanregungsstrahlung und lässt die Fluoreszenzemissionsstrahlung entlang des optischen Strahlengangs durch. Bevorzugt blockiert das Fluoreszenzfilter das Anregungslicht vollständig, während es das Fluoreszenzemissionslicht durchlässt, welches üblicherweise eine längere Wellenlänge als das Anregungslicht aufweist. Das optische Filter weist insbesondere Glas oder ein kristallines Material auf. Das optische Filter kann planar oder als Filterlinse ausgebildet sein. Prinzipiell kann anstelle des optischen Filters auch ein anderes optisches Element, wie beispielsweise eine Linse, eine Blende, ein Polarisator oder ein ähnliches optisches Element, in der Filterwechselvorrichtung und/oder der Filteraufnahme angeordnet sein.

Ein "optischer Durchgang" ist insbesondere ein ausgesparter Raum in der Filterwechselvorrichtung, durch welchen Licht durchtreten kann. Ein optischer Durchgang ist insbesondere eine durchgehende Öffnung durch die drehbare Filterplatte, weitere Bestandteile und/oder des Gehäuses der Filterwechselvorrichtung. Der optische Durchgang ist um den Mittelpunkt des Querschnittes der drehbaren Filterplatte und/oder um die optische Achse angeordnet. Der optische Durchgang erstreckt sich insbesondere entlang der optischen Achse. In Lichtausbreitungsrichtung vor und/oder in dem optischen Durchgang kann insbesondere eine Filteraufnahme und/oder ein optisches Filter angeordnet sein. Ebenso kann der optische Durchgang bei Lichtdurchtritt frei von einem angeordneten optischen Filter und/oder einer Aufnahme sein. Prinzipiell kann der optische Durchgang sämtliche Querschnittsformen aufweisen, bevorzugt ist der optische Durchgang kreisrund im Querschnitt ausgebildet.

Eine "optische Achse" ist insbesondere eine Linie, entlang der ein Grad an Rotationssymmetrie in einem optischen System existiert. Die optische Achse ist insbesondere eine imaginäre Linie, welche einen Pfad definiert, entlang dem Licht durch die Filterwechselvorrichtung und/oder die Kamera in Richtung eines Bildsensors propagiert. Vorzugsweise läuft die optische Achse durch den Krümmungsmittel des jeweils eingeschwenkten Filters und/oder eines nachgeschalteten Linsensystems und/oder Objektivsystems. Die optische Achse kann jedoch auch durch eine Linse, ein optisches Element und/oder eines der optischen Filter gebogen und/oder gelenkt werden. Der optische Strahlengang als geometrischer Verlauf von Lichtstrahlen ist insbesondere in und/oder um die optische Achse angeordnet und verläuft entlang, konvergierend und/oder dispergierend zu der optischen Achse.

Eine "drehbare Filterplatte" ist insbesondere eine Platte, welche drehbar ist und eine umlaufende Führungsnut aufweist. Die Filterplatte ist insbesondere um ihren Drehpunkt und/oder ihre Drehachse drehbar. Die Filterplatte ist insbesondere relativ zu einer Grundplatte der Filterwechselvorrichtung drehbar. Die Filterplatte kann insbesondere per Hand und/oder mittels einer Antriebseinheit und/oder einem Motor angetrieben und gedreht werden. Dazu kann beispielsweise die äußere Umfangsfläche als Kontaktfläche angetrieben werden, indem eine Antriebseinheit und/oder ein Getriebe an dieser und/oder auf diese Kontaktfläche einwirkt. Dementsprechend kann die Kontaktfläche zum Antrieb speziell ausgebildet sein, beispielsweise äußere Zahnräder, aufweisen. Die Filterplatte ist insbesondere ein flaches, ebenes Bauteil, wobei deren gegenüberliegenden Plattenflächen im Wesentlichen senkrecht zur optischen Achse ausgerichtet sind. Die Filterplatte weist insbesondere in ihrem Mittelpunkt der Plattenfläche einen optischen Durchgang durch ihre Materialdicke auf. Um die Baugröße der Filterwechselvorrichtung entlang der optischen Achse möglichst gering zu halten, weist die Filterplatte insbesondere eine geringe Materialdicke auf. Prinzipiell ist herauszustellen, dass anstelle einer Filterplatte auch ein stabförmiges Bauelement, wie beispielsweise ein Zylinder mit einer Zylinderachse ausgerichtet entlang der optischen Achse, anordenbar ist. Die Führungsnut der Filterplatte kann in eine der beiden sich gegenüberliegenden Plattenflächen eingebracht sein und somit eine geringere Nuttiefe als die Materialdicke der Filterplatte aufweisen oder die Führungsnut geht vollständig durch die Materialdicke der Filterplatte durch. Durch Drehen der Filterplatte bewegt sich das jeweilige in der Führungsnut angeordnete Führungselement in und entlang der Führungsnut, wodurch eine Rotationsbewegung des jeweiligen Filterhalters um seine Rotationsachse induziert wird. Je nach Bewegen und/oder Anordnen des jeweiligen Führungselementes in dem ersten konzentrisch ausgebildeten Abschnitt ist der Filterhalter in einer Ausgangsposition frei von dem optischen Durchgang angeordnet oder bei Bewegen und/oder Anordnen des jeweiligen Führungselementes in dem zweiten nicht-konzentrisch ausgebildeten Abschnitt wird der zugehörige Filterhalter in den optischen Durchgang ein- oder ausgeschwenkt oder befindet sich in einer Zwischenposition. Grundsätzlich ist die drehbare Filterplatte im Uhrzeigersinn und gegen den Uhrzeigersinn drehbar. Aufgrund der Ausbildung der Führungsnut mit einer geschlossenen, umlaufenden Führungsbahn ist die drehbare Filterplatte unendlich und somit mit einer beliebigen Anzahl von Umdrehungen in einer der beiden Drehrichtungen drehbar. Um die Reibungskräfte zwischen den Wänden der Führungsnut und der Außenoberfläche des jeweiligen Führungselementes zu verringern, weist die drehbare Filterplatte insbesondere ein Material mit einem niedrigen Reibungskoeffizienten auf, beispielsweise Aluminium oder ein polymeres Material, wie PTFE. Je nach Form und/oder Verlauf der Führungsnut an einer Position und/oder einem Teilabschnitt, in dem das jeweilige Führungselement angeordnet ist und/oder sich bewegt, drückt eine der beiden Innenwände oder drücken beide Innenwände der Führungsnut bei Rotation der Filterplatte gegen die Außenoberfläche des jeweiligen Führungselementes und wandeln dadurch die Rotationsbewegung der Filterplatte in eine Fortbewegung des jeweiligen Führungselementes in der Führungsnut um. Da der Filterhalter des jeweiligen Führungselementes über das Führungselement nicht nur in der Führungsnut indirekt verbunden ist, sondern auch mittels seiner Rotationsachse drehbar an der feststehenden Grundplatte verbunden ist, hängt die Schwenkbewegung des jeweiligen Filterhalters und deren Richtung von der Form und dem Verlauf der Führungsnut in dem Bereich ab, in dem sich das jeweilige Führungselement befindet.

Eine "Führungsnut" ist insbesondere ein Schlitz und/oder ein Einschnitt in ein Material und/oder in eine Oberfläche der Filterplatte. Bei einer Führungsnut handelt es sich insbesondere um eine längliche, rundumlaufende Vertiefung in der oder einen länglichen, rundumlaufenden Durchschnitt durch die Filterplatte. Die Führungsnut weist insbesondere mindestens zwei unterschiedlich ausgebildete Abschnitte in ihrer Längsrichtung auf. In dem ersten konzentrisch zur optischen Achse ausgebildeten Abschnitt weist die Führungsnut die Form eines Kreisbogens auf. In diesem ersten konzentrisch zur optischen Achse ausgebildeten Abschnitt weist die Führungsnut einen konstanten Abstand zur optischen Achse auf. Hierbei ist die optische Achse auch der Mittelpunkt der als Kreisbogen ausgebildeten Führungsnut in diesem ersten konzentrisch ausgebildeten Abschnitt. Somit ist ein Abstand und ein Radius von der optischen Achse zur kreisbogenförmig ausgebildeten Führungsnut im konzentrischen Abschnitt konstant. Folglich wird ein in diesem ersten konzentrisch zur optischen Achse ausgebildeten Abschnitt ein angeordnetes Führungselement aufgrund einer Drehung der Filterplatte gleichmäßig in diesem Abschnitt der Führungsnut weiterbewegt, wobei die beiden gegenüberliegenden Innenwände der Führungsnut gleichmäßig auf das Führungselement einwirken, sodass dem Filterhalter nicht eine Drehbewegung um seine Rotationsachse aufgezwungen wird. Dementsprechend verbleibt der Filterhalter, solange das Führungselement in diesem ersten konzentrisch zur optischen Achse ausgebildeten Abschnitt angeordnet ist, in einer Ausgangs- und/oder Ruheposition frei von dem optischen Durchgang. Somit führt dieser Filterhalter relativ zur Grundplatte keine Bewegung aus. Um eine umlaufende Führungsbahn bereitzustellen, sind ein erstes Ende und ein zweites Ende des ersten konzentrisch ausgebildeten Abschnittes mit den entsprechenden beiden Enden des zweiten nicht-konzentrisch zur optischen Achse ausgebildeten Abschnittes verbunden, sodass die mindestens zwei in der Führungsnut angeordneten Führungselemente kontinuierlich, rundumlaufend sich bei Rotation der Filterplatte in der Führungsnut bewegen können.

Unter einem "zweiten nicht-konzentrisch zur optischen Achse ausgebildeten Abschnitt" der Führungsnut wird insbesondere verstanden, dass die Führungsnut in diesem Abschnitt nicht gleich beabstandet zur optischen Achse als Mittelpunkt verläuft. Der zweite, nicht-konzentrisch ausgebildete Abschnitt weist insbesondere nicht durchweg entlang seines gesamten Verlaufes eine kreisbogenförmige Form auf, sondern ist unregelmäßig und mit unterschiedlichen Abständen und Teilabschnitten zur optischen Achse ausgebildet. Lediglich an seinen beiden Enden weist der zweite nicht-konzentrisch zur optischen Achse ausgebildete Abschnitt denselben Abstand zur optischen Achse wie der erste konzentrisch ausgebildete Abschnitt auf, um einen direkten und reibungslosen Übergang von dem zweiten nicht-konzentrisch ausgebildeten Abschnitt in den ersten konzentrisch ausgebildeten Abschnitt und umgekehrt bei der Bewegung des jeweiligen Führungselementes zu ermöglichen. Nichtsdestotrotz kann der zweite nicht-konzentrisch ausgebildete Abschnitt die Form von zwei oder mehreren aufeinanderfolgenden und/oder aneinander gereihten Kreisbögen aufweisen. Diese Kreisbögen weisen jeweils jedoch insbesondere nicht die optische Achse als ihren Mittelpunkt auf. Zudem können die jeweiligen Mittelpunkte der Kreisbogenabschnitte der zweiten nicht-konzentrisch ausgebildeten Abschnitts jeweils innerhalb der umlaufenden Führungsnut und somit zwischen der Führungsnut und der optischen Achse oder außerhalb und somit zwischen der Führungsnut und dem Außenumfang der Filterplatte liegen. Folglich kann der zweite, nicht-konzentrisch ausgebildete Abschnitt der Führungsnut aneinandergereihte kreisbogenförmige Teilabschnitte aufweisen, welche näher an der optischen Achse und/oder dem freien Durchgang liegen als der erste konzentrisch ausgebildete Abschnitt und/oder welche in einem größeren Abstand als der erste konzentrisch ausgebildete Abschnitt zur optischen Achse und/oder zum freien Durchgang angeordnet sind. Hierbei kann der maximale Außendurchmesser des jeweiligen Kreisbogensegmentes in Richtung auf die optische Achse und/oder den freien Durchgang oder in Richtung auf den Außendurchmesser der Filterplatte ausgerichtet sein. Auch können die aufeinanderfolgenden segmentbogenförmigen Teilabschnitte des zweiten nicht-konzentrisch ausgebildeten Abschnittes unterschiedliche Segmenthöhen, unterschiedliche Radien, Mittelpunktswinkel und/oder Kreisbogenlängen aufweisen. Der nicht-konzentrisch ausgebildeten Abschnitt kann auch mindestens einen linearen Teilabschnitt aufweisen.

Die Führungsnut, ihr konzentrisch ausgebildeter Abschnitt und/oder ihr nicht-konzentrisch ausgebildeter Abschnitt weisen insbesondere eine Führungsnutposition oder mehrere Führungsnutpositionen auf. Bei einer "Führungsnutposition" handelte es sich insbesondere um eine lokale Position in der Führungsnut und/oder dem jeweiligen Abschnitt, an der aufgrund des Drehens der Filterplatte die Innenwände der Führungsnut dem sich in die Führungsnutposition bewegenden oder vorliegenden Führungselement einen Druck und/oder eine Bewegungsrichtungsänderung derart aufprägen, dass der zugehörige schwenkbare Filterhalter in seiner räumlichen Ausrichtung verbleibt oder diesem eine Schwenkbewegung aufgeprägt wird. Bei einer Führungsnutposition kann es sich beispielsweise um eine neutrale Übergangsposition oder Zwischenposition des zweiten nicht-konzentrisch ausgebildeten Abschnitts oder um eine Anfangsposition oder eine Endposition des ersten konzentrisch ausgebildeten Abschnitts handeln.

Nach Durchlaufen des ersten konzentrisch ausgebildeten Abschnittes im Uhrzeigersinn kann der zweite nicht-konzentrisch ausgebildete Abschnitt am Übergang eine neutrale Übergangsposition mit einem kreisbogenförmigen Segment aufweisen, welches einen Mittelpunkt in dem innenliegend von der Führungsnut umschlossenen Bereich aufweist, sodass dieser Teilabschnitt in seinem weiteren Verlauf näher an die optische Achse heranführt, bis der zweite nicht-konzentrisch ausgebildete Abschnitt einen minimalen Abstand zur optischen Achse und/oder dem freien Durchgang in einer minimal beabstandeten Zwischenposition erreicht hat. Hierbei ist die minimal beabstandete Zwischenposition insbesondere durch ein kurzes Kreisbogensegment definiert, welches entgegengesetzt ausgerichtet mit einem Mittelpunkt außerhalb der Führungsnut und somit zwischen der Führungsnut und dem Außenumfang der Filterplatte ausgebildet ist. Beim Erreichen dieser minimal beabstandeten Zwischenposition findet aufgrund der dort wirkenden Kräfte auf das Führungselement ein Einschwenken einer Filteraufnahme des zugehörigen Filterhalters statt.

Von dieser minimal beabstandeten Zwischenposition ausgehend kann sich der weitere Verlauf der Führungsnut wieder nach außen mit einem größeren Radius biegen, sodass der Mittelpunkt dieses Teilabschnittes wiederum innerhalb des von der Führungsnut umschlossenen Bereiches liegt. Hierbei schneidet insbesondere der Verlauf des zweiten nicht-konzentrisch ausgebildeten Abschnittes in einer neutralen Übergangsposition den Radius des ersten konzentrisch ausgebildeten Abschnittes. Vorteilhaft führt er anschließend weiter in einen größeren Abstand zur optischen Achse, bis eine Führungsnutposition erreicht ist, bei welcher ein am weitesten außen liegendes Plateau mit einem größten Abstand zur optischen Achse vorliegt. Anschließend verläuft der zweite nicht-konzentrisch ausgebildete Abschnitt insbesondere in einem weiteren Teilabschnitt wieder nach innen, bis dieser den Radius des ersten konzentrisch ausgebildeten Abschnitts erreicht und in diesen übergeht.

Prinzipiell ist herauszustellen, dass die Bezeichnungen "erster" und "zweiter" Abschnitt, Filterarm, Filter und weitere Begriffe nur der Unterscheidung dienen. So hängt es beispielsweise beim Bewegen eines jeweiligen Führungselementes in der Führungsnut von der Drehrichtung im Uhrzeigersinn oder entgegen des Uhrzeigersinns ab, welcher Abschnitt als erster und welcher Abschnitt als zweiter durchlaufen wird.

Durch die asymmetrische Form des zweiten nicht-konzentrisch ausgebildeten Abschnittes, insbesondere durch Aneinanderreihung von unterschiedlichen Kreisbogensegmenten, unterschiedlich geformten Teilabschnitten, Führungsnutpositionen und/oder Rastpositionen wird gezielt eine jeweilige Bewegung des Führungselementes und somit des schwenkbaren Filterhalters vorgegeben.

Unter "Ausgangsposition" (auch "Ruheposition" genannt) wird insbesondere eine eingehaltene Position und/oder Ausrichtung des jeweiligen Filterhalters frei von dem optischen Durchgang verstanden. Hierbei ist das zugehörige Führungselementes insbesondere in der Führungsnut im ersten konzentrisch zur optischen Achse ausgebildeten Abschnitt und/oder auf dessen Radius angeordnet. Bei Bewegung des jeweiligen Führungselementes in dem ersten konzentrisch zur optischen Achse ausgebildeten Abschnitt führt der zugehörige Filterhalter keine Rotations- und/oder Schwenkbewegung um seine Rotationsachse aus. Hierbei ist der zugehörige Filterhalter in der Ausgangsposition in Längsrichtung mit seiner nach innen ausgerichteten Außenseite insbesondere mit einem Abstand benachbart zum Außendurchmesser des optischen Durchgangs angeordnet. Somit liegt der jeweilige Filterhalter in der Ruheposition insbesondere horizontal ausgerichtet unterhalb oder oberhalb des optischen Durchgangs. Die Ausgangsposition kann zusätzlich auch im nicht-konzentrisch ausgebildeten Abschnitt der Führungsnut dort vorliegen, wo der entsprechende Teilabschnitt den Radius des konzentrischen Abschnittes an einer vorgegebenen Führungsnutposition schneidet. Befindet sich ein Führungselement an dieser, insbesondere auch als neutrale Zwischenposition oder Übergangsposition bezeichneten Stelle, so ist der zugehörige Filterhalter aus dem optischen Durchgang ausgeschwenkt.

Ein "Filterhalter" ist insbesondere ein länglich ausgebildetes Element und/oder ein Arm, welcher drehbar um seine Rotationsachse auf der Grundplatte angeordnet ist. Der Filterhalter weist insbesondere eine erste Bohrung auf, in welcher die Rotationsachse angeordnet ist. Diese erste Bohrung kann durch eine Materialstärke des Filterhalters quer zu seiner Längsrichtung teilweise oder vollständig durchgehend ausgeführt sein. Eine Öffnung dieser ersten Bohrung ist insbesondere zur Grundplatte hin ausgerichtet. Die Rotationsachse des jeweiligen Filterhalters ist insbesondere im Mittelpunkt des Filterhalters angeordnet. Die Rotationsachse kann beispielsweise als Welle ausgebildet sein. Des Weiteren weist der Filterhalter an seiner Unterseite insbesondere eine zweite Bohrung auf, in welcher das Führungselement aufgenommen und/oder befestigt ist. Die Unterseite des Filterhalters ist insbesondere diejenige Seite und/oder Fläche, welche zur Führungsnut und/oder zur drehbaren Filterplatte ausgerichtet ist. Der Filterhalter weist mindestens eine Aufnahme für ein optisches Filter auf. Bevorzugt weist jeder Filterhalter zwei Aufnahmen an seinen gegenüberliegenden Enden auf.

Eine "Aufnahme" (auch "Filteraufnahme" genannt) ist insbesondere ein Hohlkörper mit einer außen teilweisen oder vollständigen Umfassung und/oder Umrandung, in welchen ein optisches Filter einsetzbar ist und welche den optischen Filter an seinem Umfang zumindest teilweise umschließt. Eine Aufnahme kann beispielsweise ein kurzer rohrförmiger Körper sein. Die Aufnahme bildet insbesondere eine Schutzhülle für das optische Filter.

Ein "Führungselement" ist insbesondere ein Element, welches einen zumindest geringfügig geringeren Außendurchmesser als der Innendurchmesser der Führungsnut aufweist, sodass das Führungselement zumindest teilweise in und/oder innerhalb der Führungsnut angeordnet ist. Das Führungselement ist insbesondere fest oder lösbar mit dem zugehörigen Filterhalter verbunden. Das Führungselement kann insbesondere direkt mit dem Filterhalter oder indirekt über ein Verbindungselement und/oder ein Kugellager mit dem Filterhalter verbunden sein. Unter "zumindest teilweise in der Führungsnut angeordnet" wird insbesondere verstanden, dass die Länge des Führungselementes angeordnet in der Führungsnut nicht zwingend sich über eine gesamte Tiefe der Führungsnut und somit dem Hohlraum der Führungsnut im Wesentlichen orthogonal zu der Fläche der Filterplatte erstrecken muss.

Eine "Grundplatte" ist insbesondere ein Bestandteil der Filterwechselvorrichtung, auf und/oder an welchem die mindestens zwei schwenkbaren Filterhalter drehbar befestigt sind. Die Grundplatte ist insbesondere frei von einer Nut. Bei einer Grundplatte kann es sich auch um ein Gehäuseteil und/oder einen Gehäusedeckel der Filterwechselvorrichtung handeln.

Eine "Kamera" (auch "Kamerakopf" genannt) ist insbesondere ein Gerät zum Empfangen von Bildlicht entlang einer optischen Achse von einem Endoskop und zum Fokussieren des empfangenen Bildlichtes auf mindestens einen Bildsensor. Neben dem mindestens einen Bildsensor kann die Kamera insbesondere eine Blende oder ein Fenster zum Durchlassen des empfangenen Bildlichtes und ein Linsensystem zum Fokussieren des Bildlichtes auf den mindestens einen Bildsensor aufweisen. Die durch mindestens einen Bildsensor aufgenommenen Bilddaten sind insbesondere elektronisch vom Kamerakopf weiter an ein Anzeigesystem und/oder an eine Bildverarbeitungseinheit übertragbar, um das endoskopische Bild für den Benutzer darzustellen. Die Kamera kann Mittel zum Erkennen des verbundenen Endoskopes und zum Verarbeiten von Algorithmen aufweisen. Ein Verbinder zum Verbinden eines Endoskops mit der Kamera kann am distalen Ende des Endoskops und/oder dem proximalen Ende des Kamerakopfs angeordnet sein. Auch kann die erfindungsgemäße Filterwechselvorrichtung selbst als Verbinder zum Verbinden eines Endoskopes mit einer Kamera ausgebildet sein.

Ein "Endoskop" ist insbesondere ein medizinisches oder industrielles Gerät zur endoskopischen Untersuchung und Betrachtung einer menschlichen oder tierischen Körperhöhle und/oder eines industriellen Hohlraums, wie einem Rohr. Das Endoskop weist insbesondere ein Handstück, einen Schaft, eine Lichtquelle, einen Lichtleiter, einen Sensor und/oder eine Kamera auf. Bei dem Endoskop handelt es sich insbesondere um ein Videoendoskop, welches eine digitale Bildaufnahme und Bildübertragung und somit eine integrierte oder verbindbare Kamera aufweist. Neben human- und tiermedizinischen Anwendungen kann ein Endoskop und/oder Videoendoskop auch zu industriellen Zwecken, beispielsweise zur Sichtprüfung in schwer zugänglichen Hohlräumen, eingesetzt werden. Bei industriellen Anwendungen wird ein Endoskop häufig als Boroskop bezeichnet.

Ein "Bildsensor" ist insbesondere ein lichtempfindliches elektronisches Bauelement, welches auf einem inneren Photoeffekt beruht. Mittels des Bildsensors wird insbesondere ein Bild oder werden mehrere Bilder aus dem Betrachtungsbereich der Bildgebungsvorrichtung aufgezeichnet und in elektronische Signale umgewandelt. Der Bildsensor weist eine Sensorebene in der Bildebene des optischen Systems, eines Linsensystems und/oder des Objektivs auf. Bei einem elektronischen Bildsensor kann es sich insbesondere um einen CCD-Sensor (charge-coupled device) oder um einen CMOS-Sensor (complementary metal oxide-semiconductor) handeln.

In einer weiteren Ausführungsform der Filterwechselvorrichtung weist der erste konzentrisch ausgebildete Abschnitt der Führungsnut eine Länge mit einer Winkelweite mit der optischen Achse als Winkelscheitel in einem Bereich von 170° bis 190°, insbesondere von 175° bis 185°, bevorzugt von 178° bis 182°, auf.

Dadurch, dass die Winkelweite und somit der Mittelpunktswinkel an der optischen Achse etwas weniger oder etwas mehr als 180° oder am meisten bevorzugt genau 180° aufweist, befindet sich zumindest ein Filterhalter der mindestens zwei Filterhalter bei einer Halbkreisdrehung in der Ausgangsposition.

Damit beide Filterhalter in einer ausgeschwenkten Ausrichtung frei von dem optischen Durchgang angeordnet sind und/oder zwischen jeder Einschwenkposition beim Drehen der Filterplatte eine nachfolgende Führungsnutposition existiert, in welcher kein Filter und/oder keine Aufnahme in den optischen Durchgang eingeschwenkt ist, kann der erste konzentrisch ausgebildete Abschnitt der Führungsnut an seinem einen Ende eine Anfangsposition und an seinem anderen Ende eine Endposition aufweisen, sodass bei einer Anordnung des Führungselementes des ersten Filterhalters in der Anfangsposition und des Führungselementes des zweiten Filterhalters in der Endposition beide Filterhalter eine Position frei von dem optischen Durchgang und/oder die jeweilige Ausgangsposition aufweisen.

Dadurch, dass nach jedem Einschwenken der Aufnahme eines Filterhalters in den optischen Durchgang bei weiterem Drehen der Filterplatte anschließend eine Position ohne Einschwenken einer Aufnahme und/oder eines Filters des entsprechenden Filterhalters in den Strahlengang erreicht wird, wird dem Benutzer der Filterwechselvorrichtung deutlich angezeigt, dass ein Wechsel zwischen dem bereits benutzten Filter und dem als nächstes zu benutzenden Filter erfolgt. Durch diese gezielte Freihaltung des optischen Durchgangs zwischen den eingeschwenkten Filtern wird ein Verwechseln von Filtern und die unbeabsichtigte Anwendung eines falschen Filters, beispielsweise bei Verwenden von unterschiedlichen Fluoreszenzfiltern, verhindert.

In einer weiteren Ausführungsform der Filterwechselvorrichtung ist die Rotationsachse des jeweiligen Filterhalters zwischen der optischen Achse und der Führungsnut oder zwischen der Führungsnut und einem Außendurchmesser der drehbaren Filterplatte angeordnet.

Da die jeweilige Rotationsachse des jeweiligen Filterhalters fest mit der Grundplatte verbunden ist, sodass der jeweilige Filterhalter drehbar gelagert ist, können durch die Anordnung der Rotationsachsen in dem von der Führungsnut umschlossenen innenliegenden Bereich und/oder in dem Bereich außen um die Führungsnut die Rotationsradien der Filterhalter festgelegt und/oder ein Überschneiden der Rotationsradien der Filterhalter beim Ein- und/oder Ausschwenken in Relation zur Geometrie und dem Verlauf der Führungsnut eingestellt werden. Durch gezieltes Platzieren der Rotationsachsen der Filterhalter über der Fläche der Grundplatte können mit einem angepassten Verlauf der Führungsnut mit ihren beiden Abschnitten auch mehr als zwei Filterhalter in der Filterwechselvorrichtung angeordnet sein.

Um die Anzahl der wechselbaren Filter zu erhöhen, kann der Filterhalter oder können die Filterhalter an zwei gegenüberliegenden Enden jeweils eine Aufnahme aufweisen und die Rotationsachse kann mittig zwischen den beiden gegenüberliegenden Enden angeordnet sein.

Somit wird bei Anordnung von zwei Filterhaltern in der Filterwechselvorrichtung das Einschwenken von vier Aufnahmen und/oder Filtern nacheinander ermöglicht. Zudem werden die Reibungskräfte reduziert und somit ein potentieller Verschleiß vermindert, da für zwei Aufnahmen und/oder Filter jeweils nur eine Rotationsachse des Filterhalters und auch nur ein Führungselement in der Führungsnut notwendig sind.

Hierbei ist es besonders vorteilhaft, dass die beiden Filter eines Filterhalter zuerst nacheinander in den optischen Durchgang und somit in den Strahlengang eingeschwenkt werden können, bevor ein Wechsel auf einen zweiten Filterhalter erfolgt. Dadurch liegen bei einem Wechsel zwischen den beiden Filtern eines Filterhalters kürzere Filterwechselzeiten vor, da der Filterhalter nur eine Links- oder Rechtsrotation um seine Rotationsachse durchführen muss, um von seinem ersten Filter auf sein zweites Filter zu wechseln oder umgekehrt.

In einer weiteren Ausführungsform der Filterwechselvorrichtung ist das Führungselement des jeweiligen Filterhalters zwischen der Rotationsachse und einer der beiden Aufnahmen, insbesondere näher an der Rotationsachse als an der jeweiligen Aufnahme, angeordnet.

Durch ein Positionieren des Führungselementes in Relation zur Rotationsachse an dem jeweiligen Filterhalter kann unter Berücksichtigung des Verlaufs der Führungsnut, in welcher das Führungselement sich bei Rotation der Filterplatte bewegt, eine definierte Rotation des Filterhalters und somit ein Einschwenken und Ausschwenken der jeweiligen Aufnahme in den optischen Durchgang realisiert werden.

Um eine definierte Einschwenkposition einer Aufnahme eines Filterhalters in den optischen Durchgang bereitzustellen, weist der zweite nicht-konzentrisch ausgebildete Abschnitt der Führungsnut vorteilhaft einen ersten Teilabschnitt mit einer maximal beabstandeten Führungsnutposition auf, welcher einen größeren Abstand zur optischen Achse als der erste konzentrisch ausgebildete Abschnitt aufweist, sodass durch Anordnung des jeweiligen Führungselementes an der maximal beabstandeten Führungsnutposition der jeweilige Filterhalter in den optischen Durchgang einschwenkbar ist.

Hierbei kann die Rotationsachse des zweiten Filterhalters auf der Grundplatte derart angeordnet sein, dass sich das zugehörige Führungselement im ersten konzentrisch ausgebildeten Abschnitt der Führungsnut befindet, sodass dieser Filterhalter in der Ausgangsposition frei von dem optischen Durchgang angeordnet ist, während aufgrund der Anordnung des ersten Führungselementes an der maximal beabstandeten Führungsnutposition der erste Filterhalter eingeschwenkt ist.

In einer weiteren Ausführungsform der Filterwechselvorrichtung weist der zweite nicht-konzentrisch ausgebildete Abschnitt der Führungsnut einen zweiten Teilabschnitt mit einer minimal beabstandeten Führungsnutposition auf, welcher einen kleineren Abstand zur optischen Achse als der erste konzentrisch ausgebildete Abschnitt aufweist, sodass bei Anordnung des jeweiligen Führungselementes an der minimal beanstandeten Führungsnutposition diejenige Aufnahme des jeweiligen Filterhalters, welche am nächsten zu dem Führungselement angeordnet ist, in den optischen Durchgang eingeschwenkt ist.

Dadurch können bei zwei Aufnahmen desjenigen Filterhalters, dessen Führungselement sich in dem zweiten nicht-konzentrischen Abschnitt der Führungsnut befindet, bei einem Durchlaufen dieses Führungselementes durch diesen zweiten nicht-konzentrischen Abschnitt nacheinander beide Aufnahmen dieses Filterhalters in den optischen Durchgang eingeschwenkt werden.

Um zwischen dem Einschwenken einer Aufnahme und/oder eines Filters an der maximal beabstandeten Führungsnutposition und dem Einschwenken der zweiten Aufnahme und/oder des zweiten Filters an der minimal beabstandeten Führungsnutposition eine eindeutige zeitliche und anwenderspezifische Trennung zu realisieren, kann zwischen dem ersten Teilabschnitt und dem zweiten Teilabschnitt des zweiten nicht-konzentrisch ausgebildeten Abschnittes ein Übergangsabschnitt mit einer Übergangsposition angeordnet sein, sodass bei Anordnung des jeweiligen Führungselementes beide Aufnahmen des jeweiligen Filterhalters aus dem optischen Durchgang ausgeschwenkt sind.

Dadurch wird dem Benutzer der Filterwechselvorrichtung eindeutig angezeigt, dass ein Wechseln der Filter erfolgt ist und folglich ein Verwechseln von zwei aufeinanderfolgenden Filtern aufgrund der zwischengeschalteten Position frei von einem Filter in dem optischen Durchgang verhindert.

In einer weiteren Ausführungsform der Filterwechselvorrichtung liegt an der Übergangsposition und an dem ersten konzentrisch ausgebildeten Abschnitt ein gleicher Abstand zur optischen Achse vor, sodass beim Bewegen des jeweiligen Führungselementes entlang der Führungsnut der jeweilige Filterhalter nach einem Anordnen in dem optischen Durchgang direkt in eine Position frei von dem optischen Durchgang und/oder in die Ausgangsposition ausschwenkbar ist.

Da der Übergangsabschnitt an der Übergangsposition aufgrund eines Wechsel von der maximal beabstandeten Führungsnutposition zu der minimal beabstandeten Führungsnutposition (oder bei einer umgekehrten Drehrichtung der Filterplatte genau umgekehrt) den gleichen Abstand von der optischen Achse wie der erste konzentrisch ausgebildete Abschnitt aufweist, liegen die beiden Führungselemente jeweils auf dem Radius des ersten konzentrisch ausgebildeten Abschnittes um die optische Achse, sodass in diesem Fall alle Aufnahmen und Filter ausgeschwenkt sind und sich beide Filterhalter in der Ausgangsposition befinden.

Um eine kompakte Anordnung der Filterhalter und/oder einen geringen Durchmesser der Filterwechselvorrichtung quer zur optischen Achse zu ermöglichen, kann der Filterhalter oder können die Filterhalter hantelförmig oder doppelkeulenförmig ausgebildet sein.

Durch die geometrische Form einer Hantel oder Doppelkeule des jeweiligen Filterhalters ist es ermöglicht, dass gleichzeitig der eine Filterhalter aus dem optischen Durchgang ausgeschwenkt und der andere Filterhalter in den optischen Durchgang eingeschwenkt wird, ohne dass die beiden Filterhalter sich trotz überschneidender Rotationsradien kollidieren. Somit wird aufgrund der Form der Filterhalter eine kompakte Anordnung der Filterhalter und eine gleichzeitige Schwenkbewegungen beider Filterhalter ermöglicht, wodurch der Durchmesser der Filterplatte, der Grundplatte und/oder der gesamten Filterwechselvorrichtung entsprechend gering ausgebildet sein kann. Hierbei kann in den beiden gegenüberliegenden Enden des hantelförmigen oder doppelkeulenförmigen Filterhalters jeweils eine Aufnahme und/oder ein Filter angeordnet sein. Die sich überschneidenden Rotationsradien können aufgrund der beiden hantelförmigen und/oder doppelkeulenförmigen Filterhalter einen Überschneidungsbereich aufweisen, welcher in seiner kleinsten Abmessung mindestens dem Außendurchmesser des jeweiligen Filterhalters im Bereich der Filteraufnahme entspricht.

In einer weiteren Ausführungsform weist die Filterwechselvorrichtung eine Rückmeldeeinrichtung bei Erreichen einer Einschwenkposition und/oder Ausschwenkposition auf.

Die Rückmeldeeinrichtung kann insbesondere derart eingerichtet sein, dass es bei Erreichen einer Führungsnutposition, einer Einschwenkposition und/oder einer Ausschwenkposition des Filterhalters eine taktile und/oder haptische Rückkopplung an den Benutzer über die drehbare Filterplatte gibt, welche von außen von dem Benutzer bedient wird. Dazu kann die Rückmeldeeinrichtung beispielsweise ein Druckstück oder mehrere Drückstücke aufweisen, welches oder welche ein festes Einrasten in jeder Führungsnut-, Einschwenk- und/oder Ausschwenkposition bewirkt oder bewirken. Das Druckstück oder die Druckstücke können wie die Rotationsachsen der Filterhalter an der Grundplatte und/oder einem Gehäusedeckel angeordnet sein. Ebenso kann die Rückmeldeeinrichtung beispielsweise haptisch gefederte Kugeln in Minibohrungen der Grundplatte aufweisen. Selbstverständlich kann die Rückmeldeeinrichtung jedoch auch statt oder ergänzend zu der haptischen und/oder taktilen Rückkopplung ein auditives oder optisches Rückkopplungssignal an den Benutzer abgegeben.

Um sowohl eine Rechtsrotation als auch eine Linksrotation des jeweiligen Filterhalters zu ermöglichen und die Filterhalter platzsparend auf und/oder an der Grundplatte anzuordnen, sind der erste konzentrisch ausgebildete Abschnitt und der zweite nicht-konzentrisch ausgebildete Abschnitt derart ausgebildet, dass die Filterhalter eine gegenläufige Drehrichtung um die jeweilige Rotationsachse und/oder jeweils einen sich überschneidenden Rotationsradius aufweisen.

Dazu kann die Führungsnut so ausgebildet sein, dass bei einer Position des jeweiligen Führungselementes mit einem größeren Abstand als der Abstand des ersten konzentrisch ausgebildeten Abschnittes von der optischen Achse und/oder an der maximal beabstandeten Führungsnutposition der zugehörige Filterhalter eine Rechtsdrehung um seine Rotationsachse ausführt. Hierbei wird das Führungselement außenliegend über die Rotationsachse gedrückt und befindet sich in einem größeren Abstand und/oder weiter außen als die Rotationsachse zur optischen Achse. Dementsprechend wird bei Erreichen des jeweiligen Führungselementes an einer Position mit einem geringeren Abstand als der Abstand des ersten konzentrisch ausgebildeten Abschnittes zur optischen Achse und/oder an der minimal beabstandeten Führungsnutposition von dem zugehörigen Filterhalter eine Linksdrehung durchgeführt, wobei dabei das Führungselement innenliegend unter die Rotationsachse gedrückt wird und sich in einem kleineren Abstand und/oder weiter innen als die Rotationsachse zur optischen Achse befindet.

In einem weiteren Aspekt der Erfindung wird die Aufgabe gelöst durch einen Kamerakopf für ein Endoskop, wobei der Kamerakopf einen Bildsensor, eine Öffnung zum Empfangen von Licht eines Bildes entlang eines optischen Pfades und ein optisches Linsensystem zur Fokussierung des Lichtes auf den Bildsensor aufweist, wobei der Kamerakopf zumindest eine zuvor beschriebene Filterwechselvorrichtung aufweist.

Somit wird ein Kamerakopf bereitgestellt, an dem oder in dem eine kompakte, platzsparend ausgebildete Filterwechselvorrichtung angeordnet ist. Im Falle der Anordnung zumindest einer Filterwechselvorrichtung direkt in dem Kamerakopf kann der Kamerakopf lösbar mit verschiedenen Arten von Endoskopen verbunden werden. Selbstverständlich kann der Kamerakopf auch zwei oder mehrere Filterwechselvorrichtungen in Reihe in einem optischen Pfad und/oder entlang der optischen Achse aufweisen.

Durch die Stapelung von zwei oder mehreren Filterwechselvorrichtungen in Reihe entweder innerhalb des Kamerakopfs oder alternativ zwischen dem proximalen Ende eines Endoskops und dem distalen Ende des Kamerakopfs können verschiedene mögliche Filtereinstellungen und -anwendungen realisiert werden, insbesondere in multispektralen Bildgebungen und/oder in einer breiten Anwendung von verschiedenen Fluorophoren in der Fluoreszenz-Bildgebung.

Um eine derzeitig verwendete Filterkonfiguration oder diese für gewünschte unterschiedliche Beobachtungsmodi anzupassen, kann der Kamerakopf eine Detektionseinheit zum Detektieren einer Identifikation des jeweiligen optischen Filters in dem optischen Pfad aufweisen. Ebenso kann der Kamerakopf eine Kontrolleinheit zum Anpassen der Drehgeschwindigkeit der Filterplatte und zum Überprüfen und/oder Anpassen des jeweiligen optischen Filters angeordnet im Strahlengang entsprechend des jeweils gewählten Betriebsmodus aufweisen.

In einem weiteren Aspekt der Erfindung wird due Aufgabe gelöst durch einen Nachrüstsatz zum Nachrüsten eines Kamerakopfes und/oder eines Endoskopes, wobei der Nachrüstsatz zumindest eine zuvor beschriebene Filterwechselvorrichtung aufweist, sodass die Filterwechselvorrichtung zwischen einem proximalen Ende des Endoskops und einem distalen Ende des Kamerakopfs anordenbar ist.

Somit wird ein Nachrüstsatz (auch als "Adapter" bezeichnet) mit mindestens einer Filterwechselvorrichtung bereitgestellt, welcher gleichzeitig als Verbinder zwischen einem bereits existierenden Endoskop und einem existierenden Kamerakopf als auch zum Ermöglichen von unterschiedlichen Beobachtungsmodi dient. Zusätzlich kann der Nachrüstsatz auch zwei oder mehrere Filterwechselvorrichtungen aufweisen, welche in Reihe zwischen dem Endoskop und dem Kamerakopf angeordnet sind oder eine Filterwechselvorrichtung kann durch eine andere Filterwechselvorrichtung zum Ermöglichen von verschiedenen Anwendungen und/oder Beobachtungsoptionen ersetzt werden.

Im Weiteren wird die Erfindung anhand von Ausführungsbeispielen näher erläutert. Es zeigen
- Figur 1: eine schematische dreidimensionale Ausschnittsansicht eines Endoskopsystems mit einem Endoskop, einem Filterwechsler und einem Kamerakopf,
- Figur 2: eine dreidimensionale Darstellung eines Filterwechslers in Seitenansicht mit einer Endoskopaufnahme und einer Kameraaufnahme,
- Figur 3: eine dreidimensionale Darstellung des Filterwechslers aus Figur 2 mit zwei Filterschwenkarmen in einer Ausgangsposition in einer Schnittdarstellung,
- Figur 4: eine stark schematische Darstellung des Filterwechslers aus Figur 3 bei geöffnetem Gehäuse und einem eingeschwenkten Filterschwenkarm,
- Figur 5: eine dreidimensionale Darstellung eines Filterschwenkarms,
- Figur 6: eine schematische dreidimensionale Darstellung eines Deckels des Filterwechslers mit den daran angeordneten Filterschwenkarmen,
- Figur 7: eine stark schematische Darstellung eines Filterrades mit einer umlaufenden Führungsnut,
- Figur 8: eine stark schematische Darstellung des Filterrades mit der Führungsnut mit Teilabschnitten und Führungsnutpositionen,
- Figur 9: eine stark schematische Darstellung des Filterwechslers mit beiden Filterschwenkarmen in der Ausgangsposition, und
- Figur 10: eine stark schematische Darstellung der Führungsnut zugehörig zu Figur 9,
- Figur 11: eine stark schematische Darstellung des Filterwechslers mit einem eingeschwenkten Filterschwenkarm und einem Filter in einem optischen Durchgang, und
- Figur 12: eine stark schematische Darstellung der Führungsnut zugehörig zu Figur 11,
- Figur 13: eine stark schematische Darstellung des Filterwechslers mit beiden Filterschwenkarmen im ausgeschwenkten Zustand, und
- Figur 14: eine stark schematische Darstellung der Führungsnut zugehörig zu Figur 13,
- Figur 15: eine stark schematische Darstellung des Filterwechslers mit dem erneut eingeschwenkten Filterschwenkarm mit dem anderen Filter im optischen Durchgang, und
- Figur 16: eine stark schematische Darstellung der Führungsnut zugehörig zu Figur 15.

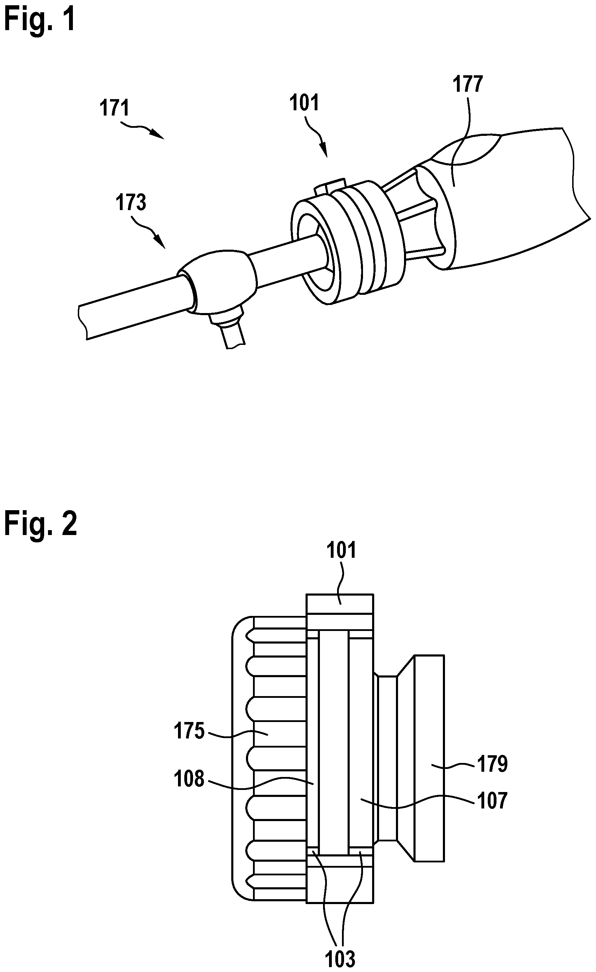

Ein Endoskopsystem 171 weist einen Kamerakopf 177, einen Filterwechsler 101 und ein Endoskop 173 auf. Der Filterwechsler 101 ist mittels einer Kameraaufnahme 179 an dem Kamerakopf 177 und mittels einer Endoskopaufnahme 175 mit dem Endoskop 173 verbunden (Figur 1 und 2). Des Weiteren weist der Filterwechsler 101 ein Gehäuse 103 mit einer als proximalen Deckel ausgebildeten Grundplatte 107 auf. Die Grundplatte 107 ist außenliegend mittels Schrauben 105 mit einem distalen Deckel 108 verbunden.

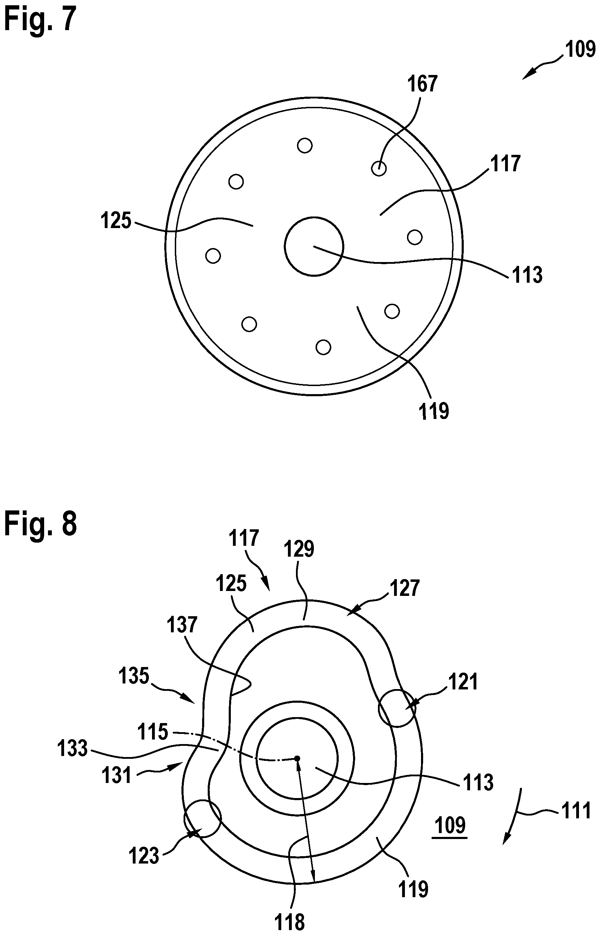

Zwischen dem distalseitigen Deckel 108 und der Grundplatte 107 ist ein drehbares Filterrad 109 angeordnet. Das Filterrad 109 ist mittels eines nicht gezeigten Motors drehbar. Die Grundplatte 107 und das Filterrad 109 weisen jeweils einen optischen Durchgang 113 um eine optische Achse 115 auf. Das drehbare Filterrad 109 weist eine Führungsnut 117 mit einem konzentrischen Führungsbahnabschnitt 119 und einem nicht-konzentrischen Führungsbahnabschnitt 125 auf. Die umlaufende Führungsnut 117 ist zwischen dem optischen Durchgang 113 und einem Außendurchmesser des Filterrades 109 angeordnet. Außen um die Führungsnut 117 herum sind gleich verteilt acht Druckstückaufnahmen 167 angeordnet (siehe Figur 7).

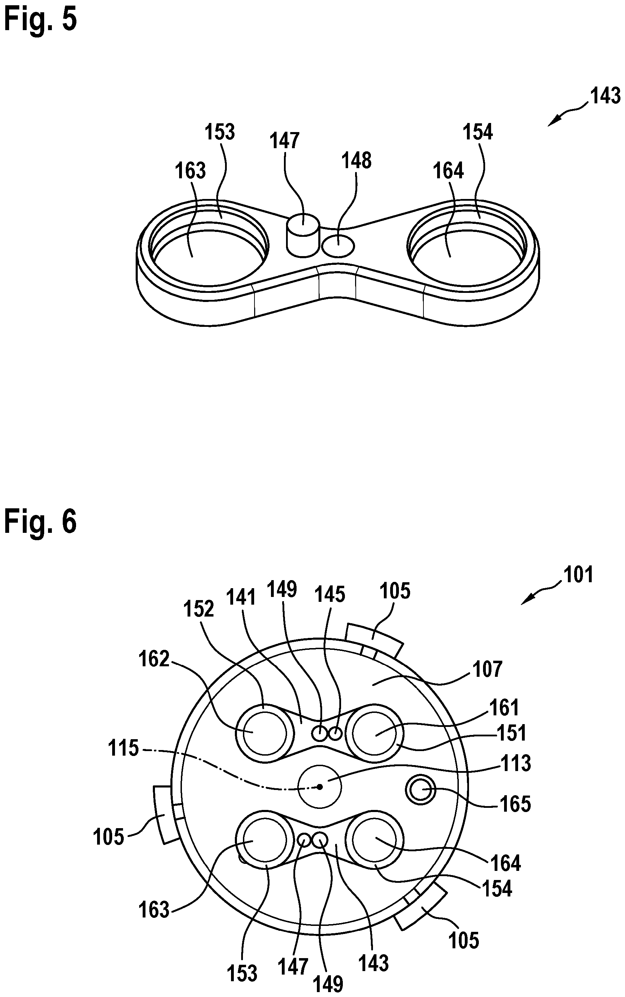

Die Grundplatte 107 ist feststehend und weist auf ihrer Seite ausgerichtet zum Filterrad 109 ein Druckstück 165 auf, welches teilweise in eine der Druckstückaufnahmen 167 aufnehmbar ist. Des Weiteren sind an dieser Seite der Grundplatte 107 ein erster Filterschwenkarm 141 und ein zweiter Filterschwenkarm 143 schwenkbar mittels jeweils einer Rotationsachse 149 befestigt. Der erste Filterschwenkarm 141 und der zweite Filterschwenkarm 143 sind mittels der jeweiligen Rotationsachsen 149 derart an der Grundplatte 107 befestigt, dass ein erster Führungsbolzen 145 des ersten Filterschwenkarms 141 und ein zweiter Führungsbolzen 147 des zweiten Filterschwenkarms 143 in der Führungsnut 117 angeordnet sind. In der Figur 6 ist eine Ausrichtung dieser beiden Filterschwenkarme 141, 143 in einer Ausgangsposition gezeigt, bei der beide Filterschwenkarme 141, 143 aus dem optischen Durchgang 113 ausgeschwenkt und parallel zueinander angeordnet sind.

Die Filterschwenkarme 141, 143 sind doppelkeulenförmig mit zwei gegenüberliegenden Enden ausgeführt. Der erste Filterschwenkarm 141 weist die Rotationsachse 149 und den ersten Führungsbolzen 145 sowie an seinen keulenförmigen Enden auf der einen Seite eine erste Filteraufnahme 151 mit einem aufgenommenen ersten Filter 161 und gegenüberliegend eine zweite Filteraufnahme 152 mit einem aufgenommenen zweiten Filter 162 auf. Wie dies beispielhaft für den zweiten Filterschwenkarm 143 in der Figur 5 gezeigt ist, weist der doppelkeulenförmige Filterschwenkarm eine durchgehende mittige Bohrung 148 zur Aufnahme der Rotationsachse 149 auf. Neben dieser Bohrung 148 ist der zweite Führungsbolzen 147 angeordnet. Das keulenförmige Ende des Filterhalters 143 weist neben dem zweiten Führungsbolzen 147 eine dritte Filteraufnahme 153 mit einem dritten Filter 163 auf, während am gegenüberliegenden zweiten keulenförmigen Ende eine vierte Filteraufnahme 154 mit einem vierten Filter 164 angeordnet ist.

Die detaillierte Ausbildung und der Verlauf der Führungsnut 117 in dem Filterrad 109 ist in Figur 8 gezeigt. Der konzentrische Führungsbahnabschnitt 119 mit einem Umfang von 180° weist einen Anfang 121 und ein gegenüberliegendes Ende 123 bei einer Rotationsrichtung 111 im Uhrzeigersinn auf (bei einer entgegengesetzten Rotation gegen den Uhrzeigersinn sind der Anfang 121 und das Ende 123 entsprechend umgekehrt). Dieser konzentrische Führungsbahnabschnitt 119 weist einen konstanten Radius 118 zur optischen Achse 115 auf. Am Ende 123 geht der konzentrische Führungsbahnabschnitt 119 in den nicht-konzentrischen Führungsbahnabschnitt 125 über. Ebenso geht der Anfang 121 des konzentrischen Führungsbahnabschnittes 119 in ein gegenüberliegendes Ende des nicht-konzentrischen Führungsbahnabschnittes 125 über.

Der nicht-konzentrische Führungsbahnabschnitt 125 weist ausgehend vom Anfang 121 des konzentrischen Führungsbahnabschnittes 119 und somit gegen die Rotationsrichtung 111 einen ersten Teilabschnitt 127 mit einer maximal beabstandeten Führungsnutposition 129 auf. Dieser erste Teilabschnitt 127 ist als ein nach außen gebogener Kreisbogenabschnitt mit einem Mittelpunktswinkel zwischen dem nicht-konzentrischen Führungsbahnabschnitt 125 und dem optischen Durchgang 113 ausgebildet. Die maximal beabstandete Führungsnutposition 129 weist einen größeren Abstand von der optischen Achse 115 als der Radius 118 des konzentrischen Führungsbahnabschnittes 119 auf. Nach dem ersten Teilabschnitt 127 schließt sich ein Übergangsabschnitt 135 entgegen der Rotationsrichtung 111 an, welcher einen größeren Kreisbogen als der erste Teilabschnitt 127 aufweist. Der Übergangsabschnitt 135 weist eine Übergangsposition 137 auf, an welcher der Übergangsabschnitt 135 den Radius 118 des konzentrischen Führungsbahnabschnittes 119 schneidet. Nachfolgend entgegen der Rotationsrichtung 111 geht der Übergangsabschnitt 135 in einen zweiten Teilabschnitt 131 über. Der zweite Teilabschnitt 131 ist als kürzerer nach innen gerichteter Kreisbogen mit einem Mittelpunktswinkel außerhalb der Führungsnut 117 gerichtet zum Außenumfang des Filterrades 109 ausgebildet. Der zweite Teilabschnitt 131 weist eine minimal beabstandete Führungsnutposition 133 auf, bei welcher ein geringster Abstand der Führungsnut 117 und des nicht-konzentrischen Führungsbahnabschnittes 125 zur optischen Achse 115 vorliegt. Dieser Abstand beträgt circa 50 % des Radius 118. Anschließend geht der zweite Teilabschnitt 131 entgegen der Rotationsrichtung 111 wieder in einem nach außen gerichteten Kreisbogen zulaufend auf den Radius 118 in das Ende 123 des konzentrischen Führungsbahnabschnittes 119 über.

Mit dem Filterwechsler 101 und dem Endoskopsystem 171 werden folgende Arbeitsgänge durchgeführt.

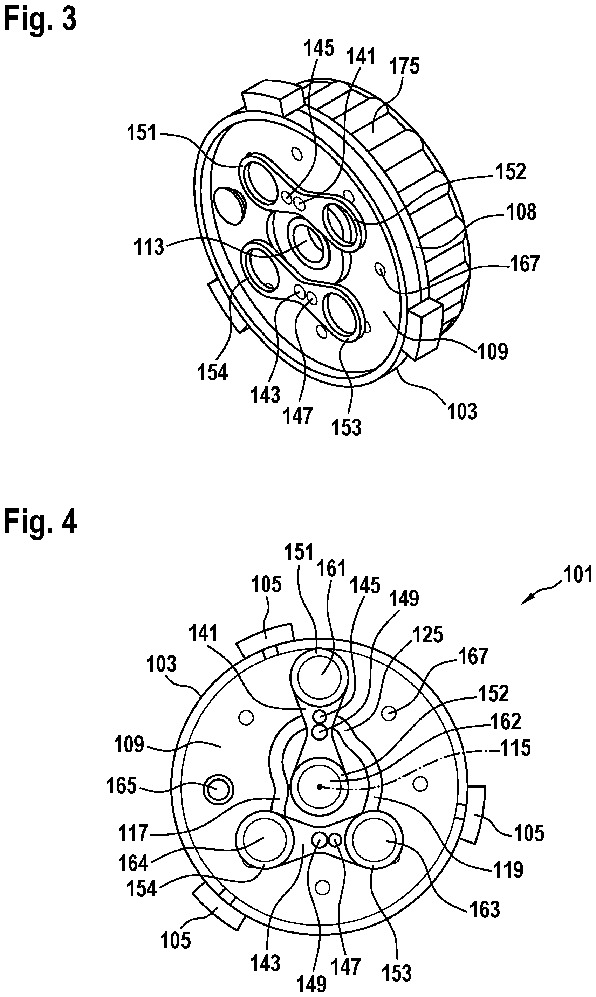

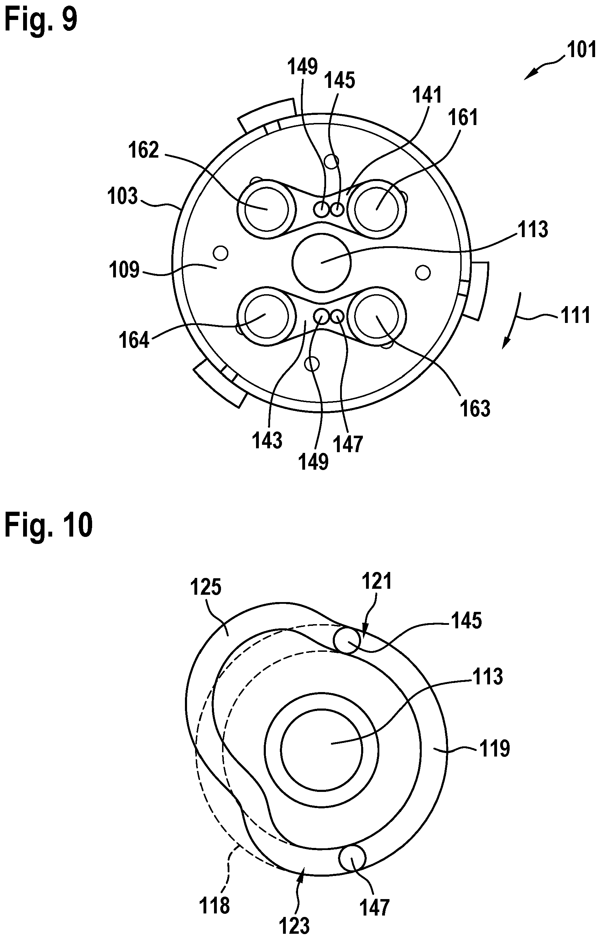

Ausgehend von einer Ausgangsposition des ersten Filterschwenkarms 141 und des zweiten Filterschwenkarms 143 liegen diese beiden Filterschwenkarme 141, 143 jeweils in einem ausgeschwenkten Zustand parallel zueinander und der optische Durchgang 113 ist frei durchgängig (siehe Figur 9 sowie die in den Figuren 3 und 6 gezeigten Ausgangspositionen). Hierbei ist der erste Führungsbolzen 145 des ersten Filterschwenkarms 141 am Anfang 121 des konzentrischen Führungsbahnabschnittes 119 angeordnet und der zweite Führungsbolzen 147 des zweiten Filterschwenkarms 143 befindet sich im konzentrischen Führungsbahnabschnitt 119 kurz vor dessen Ende 123. Somit liegen beide Führungsbolzen 145 und 147 auf dem Radius 118 und entsprechend sind die beiden Filterschwenkarme 141, 143 nicht in den optischen Durchgang 113 eingeschwenkt, sondern befinden sich in der Ausgangsposition (Figuren 9 und 10).

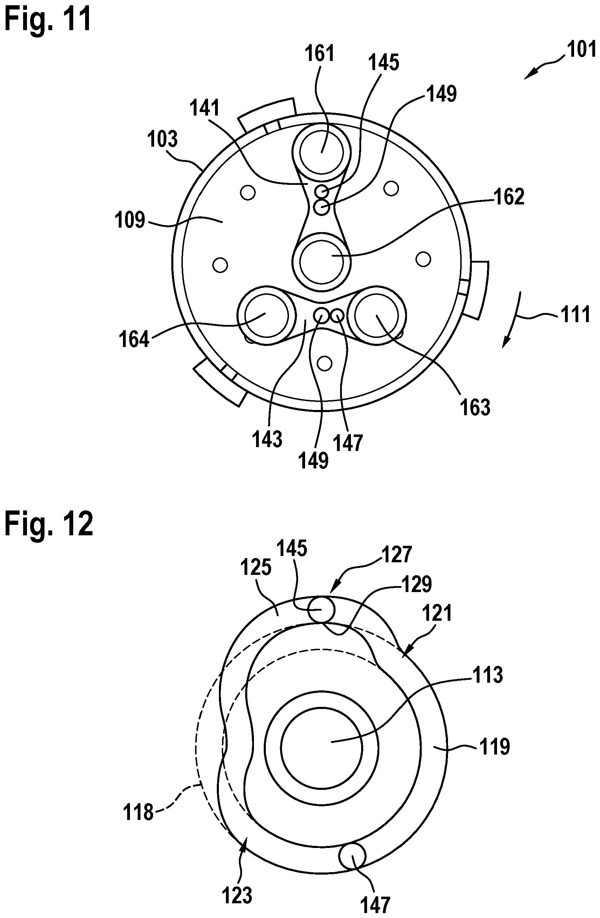

Bei Rotation des Filterrades 109 manuell durch einen Bediener oder mittels des nicht gezeigten Motors in der Rotationsrichtung 111 wird der erste Führungsbolzen 145 vom Anfang 121 des konzentrischen Führungsbahnabschnittes 119 in den ersten Teilabschnitt 127 des nicht-konzentrischen Führungsbahnabschnittes 125 geschoben, bis dieser die maximal beabstandete Führungsnutposition 129 erreicht. Aufgrund der durch die Innenwände der Führungsnut 117 in diesem ersten Teilabschnitt 127 wirkenden Kräfte auf den ersten Führungsbolzen 145 und der schwenkbaren Lagerung des ersten Filterschwenkarms 141 mittels der Rotationsachse 149 an der feststehenden Grundplatte 107 führt der erste Filterschwenkarm 141 eine Rechtsrotation aus, wobei der erste Führungsbolzen 145 senkrecht über die Rotationsachse 149 des ersten Filterschwenkarms 141 gedrückt wird und dadurch das zweite Filter 162 des ersten Filterschwenkarms 141 in den optischen Durchgang 113 eingeschwenkt wird (Figuren 11 und 12). Dieser eingeschwenkte Zustand wird dem Benutzer haptisch durch Einrasten des Druckstückes 165 in die entsprechende Druckstückaufnahme 167 angezeigt.

Währenddessen befindet sich der zweite Führungsbolzen 147 des zweiten Filterschwenkarms 143 im konzentrischen Führungsbahnabschnitt 119 und hat sich entgegen der Rotationsrichtung 111 in Richtung auf den Anfang 121 des konzentrischen Führungsbahnabschnittes 119 bewegt. Somit verbleibt der zweite Filterschwenkarm 143 in der Ausgangsposition und ist nicht in den optischen Durchgang 113 eingeschwenkt, da sich der zweite Führungsbolzen 147 weiterhin rechts neben der Rotationsachse 149 befindet (Figur 11).

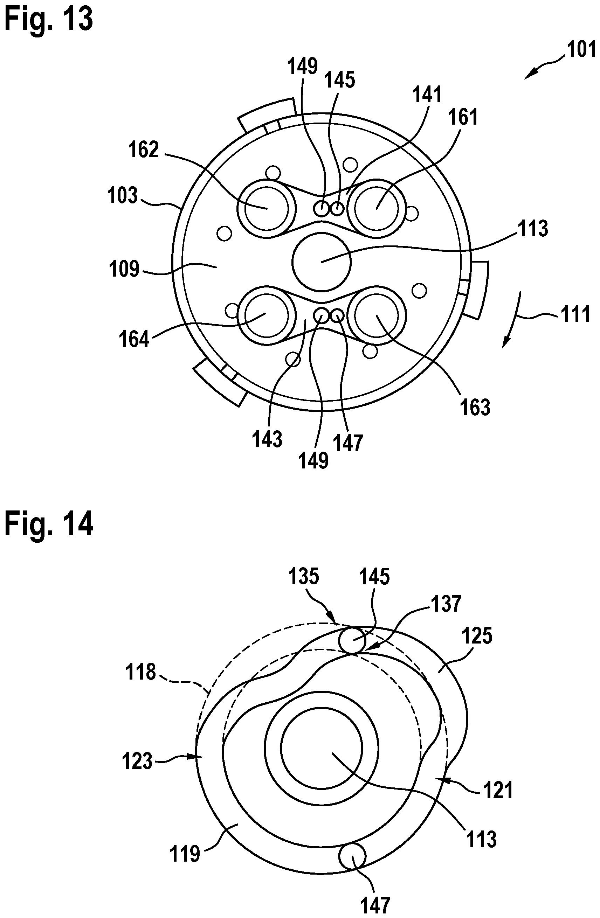

Nachdem der Benutzer das zweite Filter 162 im Strahlengang verwendet hat, dreht der Benutzer das Filterrad 109 weiter in der Rotationsrichtung 111. Dadurch wird der erste Führungsbolzen 145 entgegen der Rotationsrichtung 111 von dem ersten Teilabschnitt 127 in den Übergangsabschnitt 135 geschoben und erreicht dort die Übergangsposition 137, in welcher sich der erste Führungsbolzen 145 auf dem Radius 118 befindet. Der zweite Führungsbolzen 147 wird gleichzeitig entgegen der Rotationsrichtung 111 in dem konzentrischen Führungsbahnabschnitt 119 weiter in Richtung auf den Anfang 121 verschoben. Da beide Führungsbolzen 145, 147 nun auf dem Radius 118 liegen, befinden sich der erste Filterschwenkarm 141 und der zweite Filterschwenkarm 143 erneut in der Ausgangsposition und sind ausgeschwenkt parallel zueinander angeordnet (Figuren 13 und 14).

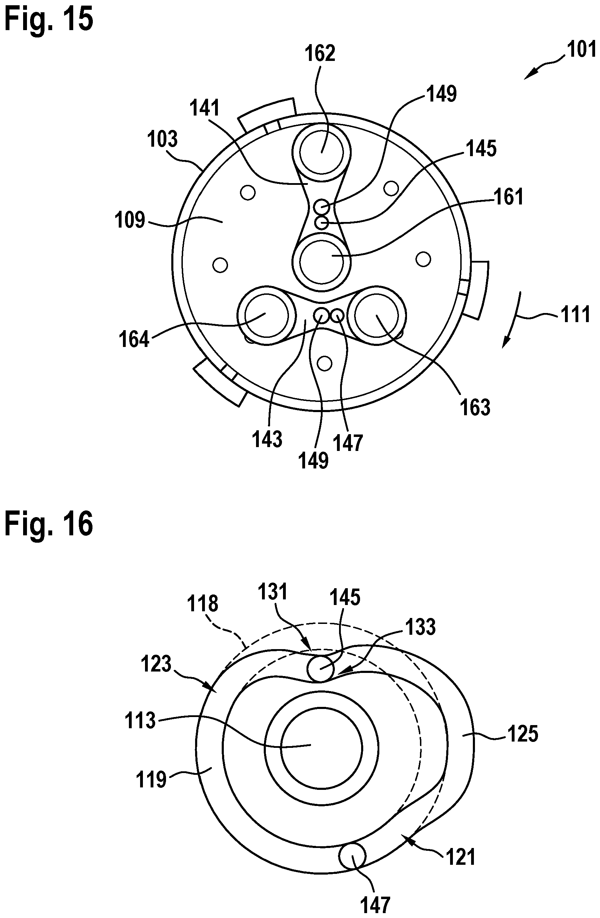

Um nun das erste Filter 161 des ersten Filterschwenkarms 141 in den optischen Durchgang 113 einzuschwenken, dreht der Benutzer anschließend das Filterrad 109 weiter in der Rotationsrichtung 111, wodurch durch die entsprechende Bewegung der Führungsnut 117 der erste Führungsbolzen 145 des ersten Filterschwenkarms 141 von der Übergangsposition 137 in den zweiten Teilabschnitt 131 des nicht-konzentrischen Führungsbahnabschnittes 125 bewegt wird, bis der erste Führungsbolzen 145 die minimal beabstandete Führungsnutposition 133 erreicht. Hierbei wird der erste Führungsbolzen 145 unterhalb der Rotationsachse 149 gedrückt, sodass der erste Filterschwenkarm 141 entsprechend eine Linksrotation ausgeführt hat und das erste Filter 161 in den optischen Durchgang 113 eingeschwenkt wird. Das Einschwenken des ersten Filters 161 wird dem Benutzer wiederum durch Einrasten des Druckstückes 165 in die entsprechende Druckstückaufnahme 167 angezeigt. Gleichzeitig wird bei der Rotation des Filterrads 109 der zweite Führungsbolzen 147 weiter in Richtung auf den Anfang 121 des konzentrischen Führungsbahnabschnittes 119 verschoben. Da der zweite Führungsbolzen 147 sich weiterhin auf dem Radius 118 des konzentrischen Führungsbahnabschnittes 119 befindet, verbleibt der zweite Filterschwenkarm 143 in der Ausgangsposition (Figuren 15 und 16).

Dreht der Benutzer nun das Filterrad 109 weiter in die Rotationsrichtung 111, so erreicht der zweite Führungsbolzen 147 den Anfang 121 des konzentrischen Führungsbahnabschnittes 119 und nimmt nacheinander bei einem weiteren Drehen in der Rotationsrichtung 111 die oben für den ersten Führungsbolzen 145 beschriebenen aufeinanderfolgenden Führungsnutpositionen im nicht-konzentrischen Führungsbahnabschnitt 125 ein, während sich der erste Bolzen 145 entsprechend innerhalb des konzentrischen Führungsbahnabschnittes 119 auf dem Radius 118 befindet und folglich ausgeschwenkt bleibt.

Optional kann der Benutzer das Filterrad 109 stattdessen auch entgegen der Rotationsrichtung 111 drehen, wobei dann der Verlauf des ersten Führungsbolzens 145 in dem nicht-konzentrischen Führungsbahnabschnitt 125 entsprechend umgekehrt zur obigen Beschreibung durchlaufen wird und der erste Filterschwenkarm 141 die entsprechenden Schwenkbewegungen umgekehrt zur oben beschriebenen Reihenfolge durchführt, während der zweite Führungsbolzen 147 wiederum in dem konzentrischen Führungsbahnabschnitt 119 auf dem Radius 118 verbleibt. Ebenso kann der Benutzer selbstverständlich in jeder Position der Führungsbolzen 145, 147 in der Führungsnut 117 beliebig von der Rotationsrichtung 111 im Uhrzeigersinn in eine entgegengesetzte Rotationsrichtung gegen den Uhrzeigersinn wechseln.

Somit wird ein Filterwechsler 101 bereitgestellt, mit dem schnell, effizient und präzise zwischen verschiedenen Filtern 161, 162, 163, 164 gewechselt werden kann und welcher mit verschiedenen Arten von Endoskopen 173 und Kameraköpfen 177 einsetzbar ist.

Obwohl eine Ausführungsvariante des Filterwechslers mittels der Figuren 1 bis 16 und der zugehörigen Beschreibung dargestellt und detailliert beschrieben ist, sind diese Darstellung und diese detaillierte Beschreibung illustrativ und beispielhaft zu verstehen. Es sind durchaus andere Ausführungsvarianten eines Filterwechslers im Sinne der Erfindung denkbar, bei welchen die Führungsnut eine asymmetrische Form des zweiten nicht-konzentrisch ausgebildeten Abschnitts aufweist und keiner der Teilabschnitte einen größeren Abstand zur optischen Achse als der erste konzentrisch ausgebildete Abschnitt aufweist.

Beispielsweise kann die asymmetrische Form durch Aneinanderreihung von unterschiedlichen Kreisbogensegmenten oder unterschiedlich geformten Teilabschnitten erzeugt werden, um die erwähnten Führungsnutpositionen zu realisieren. Insbesondere ist dabei eine Ausgestaltung der Filterhalter vorteilhaft, bei der an zwei gegenüberliegenden Enden jeweils eine Aufnahme vorgesehen ist. Beispielsweise können die Filterhalten hantelförmig oder doppelkeulenförmig ausgebildet sein, wie es beispielsweise in den Figuren dargestellt. Dadurch kann die Anzahl der wechselbaren Filter erhöht werden und es ist ermöglicht, dass gleichzeitig der eine Filterhalter aus dem optischen Durchgang ausgeschwenkt und der andere Filterhalter in den optischen Durchgang eingeschwenkt wird. Somit wird aufgrund der Form der Filterhalter eine kompakte Anordnung der Filterhalter und eine gleichzeitige Schwenkbewegungen beider Filterhalter ermöglicht.

### Bezugszeichenliste

- 101: Filterwechsler
- 103: Gehäuse
- 105: Schraube
- 107: Grundplatte/proximaler Deckel
- 108: distaler Deckel
- 109: Filterrad
- 111: Rotationsrichtung
- 113: optischer Durchgang
- 115: optische Achse
- 117: Führungsnut
- 118: Radius
- 119: konzentrischer Führungsbahnabschnitt
- 121: Anfang des konzentrischen Führungsbahnabschnitt
- 123: Ende des konzentrischen Führungsbahnabschnitt
- 125: nicht-konzentrischer Führungsbahnabschnitt
- 127: erster Teilabschnitt
- 129: maximale beabstandete Führungsnutposition
- 131: zweiter Teilabschnitt
- 133: minimal beabstandete Führungsnutposition
- 135: Übergangsabschnitt
- 137: Übergangsposition
- 141: erster Filterschwenkarm
- 143: zweiter Filterschwenkarm
- 145: erster Führungsbolzen
- 147: zweiter Führungsbolzen
- 148: Bohrung
- 149: Rotationsachse
- 151: erste Filteraufnahme
- 152: zweite Filteraufnahme
- 153: dritte Filteraufnahme
- 154: vierte Filteraufnahme
- 161: erstes Filter
- 162: zweites Filter
- 163: drittes Filter
- 164: viertes Filter
- 165: Druckstück
- 167: Druckstückaufnahme
- 171: Endoskopsystem
- 173: Endoskop
- 175: Endoskopaufnahme
- 177: Kamerakopf
- 179: Kameraaufnahme

## Patentansprüche

1. Filterwechselvorrichtung (101) für eine endoskopische Kamera, wobei die Filterwechselvorrichtung (101) eine Grundplatte (107), eine drehbare Filterplatte (109), einen optischen Durchgang (113) mit einer optischen Achse (115) und mindestens zwei schwenkbare Filterhalter (141, 143) mit jeweils mindestens einer Aufnahme (151, 152, 153, 154) für ein optisches Filter (161, 162, 163, 163) aufweist, wobei der optische Durchgang (113) um einen Mittelpunkt eines Querschnittes der drehbaren Filterplatte (109) angeordnet ist und
die drehbare Filterplatte (109) eine Führungsnut (117) mit einer umlaufenden Führungsbahn aufweist und relativ zur Grundplatte (107) drehbar ist, wobei die Filterhalter (141, 143) jeweils mittels einer Rotationsachse (149) drehbar auf der Grundplatte (107) angeordnet sind und jeweils ein Führungselement (145, 147) aufweisen, wobei
das jeweilige Führungselement (145, 147) zumindest teilweise in der Führungsnut (117) angeordnet ist, und die Führungsnut (117) einen ersten konzentrisch zur optischen Achse (115) ausgebildeten Abschnitt (119) und einen zweiten nicht-konzentrisch zur optischen Achse (115) ausgebildeten Abschnitt (125) aufweist, sodass bei einer Anordnung mindestens eines Führungselementes (145, 147) in dem ersten konzentrisch ausgebildeten Abschnitt (119) der jeweilige Filterhalter (141, 143) in einer Ausgangsposition frei von dem optischen Durchgang (113) angeordnet ist, **dadurch gekennzeichnet, dass** der zweite nicht-konzentrisch ausgebildete Abschnitt (125) der Führungsnut (117) einen ersten Teilabschnitt (127) mit einer maximal beabstandeten Führungsnutposition (129) aufweist, welcher einen größeren Abstand zur optischen Achse (115) als der erste konzentrisch ausgebildete Abschnitt (119) aufweist,
sodass bei Anordnung des jeweiligen Führungselementes (145, 147) an der maximal beabstandeten Führungsnutposition (129) der jeweilige Filterhalter (141, 143) in den optischen Durchgang (113) eingeschwenkt ist.

2. Filterwechselvorrichtung (101) nach Anspruch 1, **dadurch gekennzeichnet, dass** der erste konzentrisch ausgebildete Abschnitt (119) der Führungsnut (117) eine Länge mit einer Winkelweite mit der optischen Achse (115) als Winkelscheitel in einem Bereich von 170° bis 190°, insbesondere von 175° bis 185°, bevorzugt von 178° bis 182° aufweist.

3. Filterwechselvorrichtung nach Anspruch 1 oder 2, **dadurch gekennzeichnet, dass** der erste konzentrisch ausgebildete Abschnitt (119) der Führungsnut (117) an seinem einen Ende (121) eine Anfangsposition und an seinem anderen Ende (123) eine Endposition aufweist, sodass bei einer Anordnung des Führungselementes (145) des ersten Filterhalters (141) in der Anfangsposition und des Führungselementes (147) des zweiten Filterhalters (143) in der Endposition beide Filterhalter (141, 143) eine Position frei von dem optischen Durchgang (113) und/oder die jeweilige Ausgangsposition aufweisen.

4. Filterwechselvorrichtung (101) nach einem der vorherigen Ansprüche, **dadurch gekennzeichnet, dass** die Rotationsachse (149) des jeweiligen Filterhalters (141, 143) zwischen der optischen Achse (115) und der Führungsnut (117) oder zwischen der Führungsnut (117) und einem Außendurchmesser der drehbaren Filterplatte (109) angeordnet ist.

5. Filterwechselvorrichtung (101) nach einem der vorherigen Ansprüche, **dadurch gekennzeichnet, dass** der Filterhalter oder die Filterhalter (141, 143) an zwei gegenüberliegenden Enden jeweils eine Aufnahme (151, 152, 153, 154) aufweist oder aufweisen und die Rotationsachse (149) mittig zwischen den beiden gegenüberliegenden Enden angeordnet ist.

6. Filterwechselvorrichtung (101) nach Anspruch 5, **dadurch gekennzeichnet, dass** das Führungselement (145, 147) des jeweiligen Filterhalters (141, 143) zwischen der Rotationsachse (149) und einer der beiden Aufnahmen (151, 152, 153, 154), insbesondere näher an der Rotationsachse (149) als an der jeweiligen Aufnahme (151, 152, 153, 154), angeordnet ist.

7. Filterwechselvorrichtung (101) nach einem der vorherigen Ansprüche, **dadurch gekennzeichnet, dass** der zweite nicht-konzentrisch ausgebildeten Abschnitt (125) der Führungsnut (117) einen zweiten Teilabschnitt (131) mit einer minimal beabstandeten Führungsnutposition (133) aufweist, welcher einen kleineren Abstand zur optischen Achse (115) als der erste konzentrisch ausgebildete Abschnitt (119) aufweist, sodass bei Anordnung des jeweiligen Führungselementes (145, 147) an der minimal beabstandeten Führungsnutposition (133) diejenige Aufnahme (151, 152, 153, 154) des jeweiligen Filterhalters (141, 143), welche am nächsten zu dem Führungselement (145, 147) angeordnet ist, in den optischen Durchgang (113) eingeschwenkt ist.

8. Filterwechselvorrichtung (101) nach Anspruch 7, **dadurch gekennzeichnet, dass** zwischen dem ersten Teilabschnitt (127) und dem zweiten Teilabschnitt (131) des zweiten nicht-konzentrisch ausgebildeten Abschnittes (125) ein Übergangsabschnitt (135) mit einer Übergangsposition (137) angeordnet ist, sodass bei Anordnung des jeweiligen Führungselementes (145, 147) beide Aufnahmen (151, 152, 153, 154) des jeweiligen Filterhalters (141, 143) aus dem optischen Durchgang (113) ausgeschwenkt sind.

9. Filterwechselvorrichtung (101) nach Anspruch 8, **dadurch gekennzeichnet, dass** an der Übergangsposition (137) und an dem ersten konzentrisch ausgebildeten Abschnitt (119) ein gleicher Abstand zur optischen Achse (115) vorliegt, sodass beim Bewegen des jeweiligen Führungselementes (145, 147) entlang der Führungsnut (117) der jeweilige Filterhalter (141, 143) nach einem Anordnen in dem optischen Durchgang (113) direkt in eine Position frei von dem optischen Durchgang (113) und/oder in die Ausgangsposition ausschwenkbar ist.

10. Filterwechselvorrichtung (101) nach einem der vorherigen Ansprüche, **dadurch gekennzeichnet, dass** der Filterhalter (141, 143) oder die Filterhalter (141, 143) hantelförmig oder doppelkeulenförmig ausgebildet ist oder sind.

11. Filterwechselvorrichtung (101) nach einem der vorherigen Ansprüche, **dadurch gekennzeichnet, dass** Filterwechselvorrichtung (101) eine Rückmeldeeinrichtung (165, 167) bei Erreichen einer Führungsnutposition, Einschwenkposition und/oder Ausschwenkposition aufweist.

12. Filterwechselvorrichtung (101) nach einem der vorherigen Ansprüche, **dadurch gekennzeichnet, dass** der erste konzentrisch ausgebildete Abschnitt (119) und der zweite nicht-konzentrisch ausgebildete Abschnitt (125) derart ausgebildet sind, dass die Filterhalter (141, 143) eine gegenläufige Drehrichtung um die jeweilige Rotationsachse (14) und/oder jeweils einen sich überschneidenden Rotationsradius aufweisen.

13. Kamerakopf (177) für eine Endoskop (173), wobei der Kamerakopf (177) einen Bildsensor, eine Öffnung zum Empfangen von Licht eines Bildes entlang eines optischen Pfades und ein optisches Linsensystem zum Fokussieren des Lichtes auf den Bildsensor aufweist, **dadurch gekennzeichnet, dass** der Kamerakopf (177) zumindest eine Filterwechselvorrichtung (101) nach einem der Ansprüche 1 bis 12 aufweist.

14. Nachrüstsatz zum Nachrüsten eines Kamerakopfes und/oder eines Endoskopes, **dadurch gekennzeichnet, dass** der Nachrüstsatz zumindest eine Filterwechselvorrichtung (101) nach einem der Ansprüche 1 bis 12 aufweist, sodass die Filterwechselvorrichtung (101) zwischen einem proximalen Ende des Endoskopes (173) und einem distalen Ende des Kamerakopfes (177) anordenbar ist.
